# EUROPÄISCHE PATENTSCHRIFT

(11) **EP 2 976 031 B1**
(45) Veröffentlichungstag und Bekanntmachung des Hinweises auf die Patenterteilung: **12.09.2018**
(21) Anmeldenummer: 14711960.6
(22) Anmeldetag: 19.03.2014
(51) Int. Cl.: A61B 17/70

(54) **WIRBELSÄULENSTABILISIERUNGSSYSTEM UND CHIRURGISCHES BEFESTIGUNGSELEMENT FÜR EIN WIRBELSÄULENSTABILISIERUNGSSYSTEM**
VERTEBRAL COLUMN-STABILIZING SYSTEM AND SURGICAL FASTENING ELEMENT FOR A VERTEBRAL COLUMN-STABILIZING SYSTEM
SYSTÈME DE STABILISATION DE LA COLONNE VERTÉBRALE ET ÉLÉMENT DE FIXATION CHIRURGICALE POUR UN SYSTÈME DE STABILISATION DE LA COLONNE VERTÉBRALE

(30) Priorität: 22.03.2013 DE 102013102976; 15.07.2013 DE 102013107498
(43) Veröffentlichungstag der Anmeldung: 27.01.2016
(73) Patentinhaber: Aesculap AG, 78532 Tuttlingen (DE)
(72) Erfinder: LINDNER, Stephan, 78573 Wurmlingen (DE); KRÜGER, Sven, 78647 Trossingen (DE)
(74) Vertreter: Hoeger, Stellrecht & Partner Patentanwälte mbB
(86) Internationale Anmeldenummer: PCT/EP2014/055468
(87) Internationale Veröffentlichungsnummer: WO 2014/147104

(56) Entgegenhaltungen:
- EP-A1- 1 891 904
- WO-A1-96/21396
- DE-U1- 9 403 231
- DE-U1- 29 606 468
- FR-A1- 2 624 720
- FR-A1- 2 829 014
- US-A- 5 667 508
- US-A1- 2009 062 865

## Beschreibung

Die vorliegende Erfindung betrifft ein chirurgisches Befestigungselement für ein mindestens zwei Befestigungselemente und mindestens ein Verbindungselement umfassendes Wirbelsäulenstabilisierungssystem, welches Befestigungselement einen Befestigungsabschnitt, einen Halteabschnitt mit einer Verbindungselementaufnahme und ein am Halteabschnitt festlegbares Fixierelement zum Festlegen des Verbindungselements in der Verbindungselementaufnahme umfasst, wobei das Fixierelement ein Niederhalteelement zum Niederhalten des Verbindungselements in die Verbindungselementaufnahme trägt.

Ferner betrifft die vorliegende Erfindung ein Wirbelsäulenstabilisierungssystem umfassend mindestens zwei chirurgische Befestigungselemente und mindestens ein Verbindungselement, wobei mindestens eines der mindestens zwei chirurgischen Befestigungselemente einen Befestigungsabschnitt, einen Halteabschnitt mit einer Verbindungselementaufnahme und ein am Halteabschnitt festlegbares Fixierelement zum Festlegen des Verbindungselements in der Verbindungselementaufnahme umfasst, wobei das Fixierelement ein Niederhalteelement zum Niederhalten des Verbindungselements in die Verbindungselementaufnahme trägt.

Chirurgische Befestigungselemente für ein mindestens zwei Befestigungselemente und mindestens ein Verbindungselement umfassendes Wirbelsäulenstabilisierungssystem, welches Befestigungselement einen Befestigungsabschnitt, einen Halteabschnitt mit einer Verbindungselementaufnahme und ein am Halteabschnitt festlegbares Fixierelement zum Festlegen des Verbindungselements in der Verbindungselementaufnahme umfasst sowie Wirbelsäulenstabilisierungssysteme umfassend mindestens zwei chirurgische Befestigungselemente und mindestens ein Verbindungselement, wobei mindestens eines der mindestens zwei chirurgischen Befestigungselemente einen Befestigungsabschnitt, einen Halteabschnitt mit einer Verbindungselementaufnahme und ein am Halteabschnitt festlegbares Fixierelement zum Festlegen des Verbindungselements in der Verbindungselementaufnahme umfasst sind beispielsweise aus der EP 2 266 483 A1 bekannt. Die bekannten Befestigungselemente, welche insbesondere in Form von Befestigungsschrauben, beispielsweise Pedikelschrauben, ausgebildet sein können, werden zur Stabilisierung der Wirbelsäule in benachbarten Wirbelkörpern eingebracht und über ein oder mehrere Verbindungselemente miteinander verbunden. Die Verbindungselemente können stab- und/oder plattenförmig ausgebildet sein, so dass auf diese Weise eine definierte Verbindung zwischen benachbarten Wirbelkörpern einstellbar ist. Selbstverständlich können die Wirbelsäulenstabilisierungssysteme auch noch weitere Befestigungselemente und Verbindungselemente umfassen, so dass nicht nur zwei, sondern auch zwei, drei oder mehr Bewegungssegmente der Wirbelsäule miteinander gekoppelt und stabilisiert werden können.

Ein Problem insbesondere bei einer minimalinvasiven Implantation derartiger Wirbelsäulensysteme ist es, die Verbindungselemente in die Verbindungselementaufnahme einzubringen und anschließend das in der Regel eher kleine Fixierelement einzusetzen. Hierfür wird zum leichteren Einsetzen des Fixierelements das Verbindungselement üblicherweise in die Verbindungselementaufnahme hineingedrückt.

Bei bekannten System besteht das Einbringen der Befestigungselemente sowie das Festlegen der Verbindungselemente prinzipiell aus mehreren Schritten: das Befestigungselement einbringen und verankern, das Verbindungselement in die Verbindungselement in die Verbindungselementaufnahme einsetzen, das Verbindungselement in die Verbindungselementaufnahme hineindrücken und anschließend das Fixierelement einführen und das Verbindungselement unbeweglich an der Verbindungselementaufnahme mit dem Fixierelement fixieren. Das Drücken des Verbindungselements in die Verbindungselementaufnahme hinein hat insbesondere die Aufgabe, das Verbindungselement in eine definierte Endlage in der Verbindungselementaufnahme zu bringen und dort zu halten, so dass das Fixierelement eingeführt und vorzugsweise kraftfrei am Halteabschnitt festgelegt werden kann, um das Verbindungselement zu fixieren.

Bekannt ist insbesondere, externe Hülsen zum Drücken des Verbindungselements in die Verbindungselementaufnahme hinein zu nutzen. Allerdings hat dies den Nachteil, dass hierfür ein Hautschnitt deutlich vergrößert werden muss. Alternativ bekannt sind auch Befestigungselemente mit verlängerten Halteabschnitten, die nach dem Festlegen des Fixierelements teilweise entfernt werden können beziehungsweise müssen, beispielsweise durch Abrechen von als Führungen dienenden Verlängerungen am Halteabschnitt des Fixierelements. Beide Varianten haben jedoch Nachteile, die den Aufwand beim chirurgischen Eingriff erhöhen und insbesondere die Operationszeit verlängern.

Aus der EP 1 891 904 A1 ist eine Knochenverankerungsvorrichtung bekannt. Wirbelsäulenstabilisierungssysteme sind in der FR 2 829 014 A1 beschrieben. Die US 2009/0062865 A1 offenbart orthopädische Rückhaltesysteme. Aus der US 5,667,508 ist eine einstückige Sicherungskappe für eine Pedikelschraube bekannt. Ein Wirbelsäulenfixateur ist in der DE 296 06 468 U1 beschrieben. Die FR 2 624 720 A1 offenbart eine Wirbelsäulenosteosynthesevorrichtung. Aus der DE 94 03 231 U1 sind chirurgische Implantate bekannt. Ferner ist in der WO 96/21396 A1 ein Wirbelsäulenfixateur beschrieben.

Es ist daher insbesondere eine Aufgabe der vorliegenden Erfindung, die Implantation des Wirbelsäulenstabilisierungssystems der eingangs beschrieben Art zu vereinfachen.

Diese Aufgabe wird bei einem chirurgischen Befestigungselement der eingangs beschriebenen Art erfindungsgemäß insbesondere dadurch gelöst, dass das Fixierelement eine Sollbruchstelle aufweist, welche einen distalen Fixierelementabschnitt und einen proximalen Fixierelementabschnitt trennt, und dass das Niederhalteelement am proximalen Fixierelementabschnitt angeordnet ist.

Das Fixierelement des chirurgischen Befestigungselements so auszubilden, dass dieses beim Einbringen gleichzeitig auch das Verbindungselement in die Verbindungselementaufnahme hineindrücken und darin halten kann, ermöglicht es, auf den bislang separaten Operationsschritt, nämlich das Verbindungselement in die Verbindungselementaufnahme zu drücken und in dieser zu halten, komplett wegzulassen. Erfindungsgemäß kann bereits mit dem Einbringen des Fixierelements das Verbindungselement in die Verbindungselementaufnahme hineingedrückt werden. Wegen des wegfallenden Operationsschritts kann die Dauer des chirurgischen Eingriffs insgesamt verkürzt und zudem der Eingriff vereinfacht werden. Ferner kann es dabei insbesondere günstig sein, wenn in einer Verbindungsstellung, in welcher das Fixierelement und das Niederhalteelement gekoppelt sind beziehungsweise das Fixierelement das separat von diesem ausgebildete Niederhalteelement trägt, ein distales Ende des Niederhalteelements über ein distales Ende des Fixierelements vorsteht. So kann das Niederhalteelement das Verbindungselement in die Verbindungselementaufnahme drücken, und zwar insbesondere ohne dass das Fixierelement, insbesondere ein distaler Fixierelementabschnitt desselben, mit einem Innengewinde des Halteabschnitts in Eingriff steht. Ferner ist es vorteilhaft, dass das Fixierelement eine Sollbruchstelle aufweist, welche einen distalen Fixierelementabschnitt und einen proximalen Fixierelementabschnitt trennt. So kann das Fixierelement insgesamt einfach handhabbar sein, wobei nur ein Teil desselben letztendlich am Halteabschnitt verbleiben muss, um das Verbindungselement dort unbeweglich festzulegen.

Eine besonders einfache Konstruktion des Befestigungselements lässt sich insbesondere dadurch erreichen, dass das Fixierelement hülsenförmig oder im Wesentlichen hülsenförmig ausgebildet ist und eine eine Längsachse des Fixierelements konzentrisch umgebende Fixierelementwand umfasst. Dies ermöglicht es insbesondere, mit einem Werkzeug beispielsweise durch das Fixierelement hindurch direkt auf ein distales Ende des Fixierelements zuzugreifen, um es am Halteabschnitt festzulegen.

Günstigerweise umfasst das das Fixierelement einen in distaler Richtung wirkenden Anschlag für das Niederhalteelement. Auf diese Weise kann eine Bewegung des Niederhalteelements relativ zum Fixierelement in proximaler Richtung begrenzt werden.

Ein besonders einfacher konstruktiver Aufbau ergibt sich, wenn der Anschlag eine in distaler Richtung weisende Anschlagfläche umfasst, an welcher das Niederhalteelement anliegt, insbesondere in der Verbindungsstellung.

Vorzugsweise ist das Fixierelement in Form einer Fixierschraube ausgebildet, welche ein Außengewinde aufweist, welches sich ausgehend von einem distalen Ende der Fixierschraube in proximaler Richtung erstreckt, wobei das Außengewinde korrespondierend zu einem am Halteabschnitt ausgebildeten Innengewinde ausgebildet ist. Diese Ausgestaltung ermöglicht es insbesondere, das Fixierelement in das Innengewinde des Halteabschnitts einzuschrauben. So kann das Verbindungselement auf einfache und sichere Weise an der Verbindungselementaufnahme festgelegt werden.

Besonders einfach wird die Ausgestaltung des Befestigungselements, wenn die Sollbruchstelle gebildet wird durch eine bezogen auf die Längsachse umlaufende oder im Wesentlichen umlaufende, in der Fixierelementwand ausgebildete Vertiefung. Insbesondere kann die Vertiefung eine keilförmige Ringnut sein.

Die Herstellung des Befestigungselements wird besonders einfach, wenn die umlaufende Vertiefung von der Längsachse weg weisend ausgebildet ist. So lässt sie sich beispielsweise in einem letzten Schritt der Herstellung des Fixierelements ausbilden.

Vorteilhaft ist es, wenn der distale Fixierelementabschnitt einen distalen Außengewindeabschnitt aufweist und wenn der proximale Fixierelementabschnitt einen proximalen Außengewindeabschnitt aufweist. Diese Ausgestaltung ermöglicht es, sowohl den distalen Fixierelementabschnitt als auch den proximalen Fixierelementabschnitt gemeinsam und/oder jeweils getrennt mit korrespondieren Innengewindeabschnitten an Instrumenten und/oder dem Halteabschnitt zu verschrauben.

Vorzugsweise umfasst das Fixierelement einen Kopf, welcher den Anschlag bildet oder umfasst. So lässt sich der Anschlag auf besonders einfache Weise ausbilden.

Günstig ist es, wenn der Kopf einen Außendurchmesser aufweist, welcher größer ist als ein Außendurchmesser eines sich distalseitig an den Kopf anschließenden Schafts. Auf diese Weise lässt sich der Anschlag besonders einfach realisieren.

Insbesondere für eine Positionierung und Lagerung des Niederhalteelements am Fixierelement ist es vorteilhaft, wenn sich an den Kopf distalseitig ein gewindefreier Schaftabschnitt anschließt und wenn sich das Außengewinde distalseitig an den gewindefreien Schaftabschnitt anschließt.

Für die Handhabung des Befestigungselements ist es günstig, wenn das Niederhalteelement am Fixierelement rotierbar gelagert ist. Beispielsweise kann so das Niederhalteelement seine Position beziehungsweise Rotationsstellung relativ zum Halteabschnitt beibehalten und gleichzeitig das Fixierelement in axialer Richtung verschraubt werden.

Ferner ist es vorteilhaft, wenn das Niederhalteelement am Fixierelement in axialer Richtung unbeweglich oder im Wesentlichen unbeweglich gehalten ist. So kann mit einer Bewegung des Fixierelements in Richtung auf den Halteabschnitt hin das Niederhalteelement gleichzeitig das Verbindungselement in die Verbindungselementaufnahme hineindrücken und in dieser halten.

Besonders einfach herstellbar wird das Befestigungselement, wenn das Niederhalteelement hülsenförmig oder im Wesentlichen hülsenförmig ausgebildet ist. So kann es insbesondere das Fixierelement umgeben, vorzugsweise distalseitig des Kopfs, wenn das Fixierelement einen solchen aufweist. Insbesondere kann ein Außendurchmesser des Niederhalteelements an einen Außendurchmesser des Kopfs angepasst sein, so dass der Kopf einen maximalen Außendurchmesser einer aus dem Fixierelement und dem Niederhalteelement gebildeten Fixiereinheit aufweist.

Günstig ist es, wenn das Niederhalteelement bezogen auf eine Längsachse zwei einander diametral gegenüberliegende Ausnehmungen aufweist. Dies ermöglicht es, dass beispielsweise das Außengewinde des Fixierelements im Bereich der Ausnehmungen mit einem Innengewinde eines Implantationsinstruments oder dem Innengewinde des Halteabschnitts zusammenwirken kann.

Besonders einfach ausbilden lässt sich das Niederhalteelement, wenn die Ausnehmungen in Form von Schlitzen ausgebildet sind, welche sich ausgehend von einem distalen Ende des Niederhalteelements in proximaler Richtung erstrecken. Ferner ermöglicht es eine solche Ausgestaltung, die verbleibenden Lappen am Niederhalteelement von der Längsachse weg und auf die Längsachse hin zu verschwenken.

Um das Niederhalteelement in definierter und sicherer Weise am Fixierelement zu lagern, ist es günstig, wenn das Niederhalteelement einen Haltering umfasst, welcher zwei in distaler Richtung abstehende, einander diametral gegenüberliegende Niederhaltevorsprünge trägt. Beispielsweise kann der Haltering des Niederhalteelements in sich geschlossen ausgebildet sein und insbesondere über das Fixierelement von distal her kommend geschoben werden, so dass er proximalseitig am Anschlag, welcher insbesondere am Kopf des Fixierelements ausgebildet sein kann, anschlägt.

Vorzugsweise bildet der Haltering ein proximales Ende des Niederhalteelements. So können insbesondere distale Enden der Niederhaltevorsprünge mit dem in der Verbindungselementaufnahme zu positionierenden Verbindungselement in Eingriff gebracht werden.

Gemäß einer weiteren bevorzugten Ausführungsform der Erfindung kann vorgesehen sein, dass die Niederhaltevorsprünge durch die zwei einander diametral gegenüberliegenden Ausnehmungen voneinander getrennt sind. Wie bereits erwähnt, können so die Niederhaltevorsprünge mit ihren distalen Enden in Richtung auf die Längsachse hin und/oder von dieser weg bei entsprechender Ausgestaltung und Wahl des Materials, aus dem das Niederhalteelement ausgebildet ist, ausschwenken und/oder ausfedern.

Um eine möglichst große Anlagefläche des Niederhalteelements beim Anliegen an einem Verbindungselement zu erreichen, insbesondere wenn letzteres stabförmig mit rundem Querschnitt ausgebildet ist, ist es vorteilhaft, wenn distale Enden der Niederhaltevorsprünge in distaler Richtung weisend konkav gekrümmt sind.

Um eine Positionierung des Niederhalteelements am Fixierelement zu optimieren, insbesondere auch eine Position der Niederhaltevorsprünge in Umfangsrichtung, ist es vorteilhaft, wenn die Niederhaltevorsprünge in Umfangsrichtung weisende, seitlich abstehende Führungsvorsprünge tragen. Diese umgreifen somit das Fixierelement noch etwas weiter als die Niederhaltevorsprünge selbst.

Um das Einführen des Fixierelements und insbesondere eine Ausrichtung desselben in Umfangsrichtung zu verbessern, ist es günstig, wenn die Führungsvorsprünge in distaler Richtung weisende, geneigte Aufgleitflächen aufweisen. Auf diese Weise kann sich beim Einführen des Fixierelements, welches das Niederhalteelement trägt, das Niederhalteelement auf einfache Weise beim Einführen automatisch in diejenige Rotationsstellung ausrichten, welche erforderlich ist, damit ein distales Ende des Niederhalteelements mit dem Verbindungselements in Kontakt treten kann.

Vorteilhaft ist es, wenn in einer Verbindungsstellung, in welcher das Niederhalteelement mit dem Fixierelement gekoppelt ist, ein proximales Ende des Halterings am Anschlag anliegt. Wird beispielsweise das Fixierelement mittels eines an seinem Kopf angreifenden Einbringwerkzeugs, insbesondere eines Schraubendrehers, in distaler Richtung bewegt, so wird gleichzeitig auch das Niederhalteelement in distaler Richtung mitbewegt.

Um eine möglichst kompakte Bauform zu erreichen, ist es günstig, wenn ein Außendurchmesser des Halterings einem Außendurchmesser des Kopfs entspricht oder im Wesentlichen entspricht.

Gemäß einer weiteren bevorzugten Ausführungsform der Erfindung kann eine Rastverbindung zum lösbaren Verbinden des Fixierelements und des Niederhalteelements in einer Verbindungsstellung vorgesehen sein.

Ein besonders einfacher Aufbau des Befestigungselements lässt sich insbesondere dadurch erreichen, dass die Rastverbindung mindestens einen Rastvorsprung und mindestens eine mit diesem zusammenwirkende Rastausnehmung umfasst. Die Rastausnehmung kann grundsätzlich sowohl am Niederhalteelement als auch am Fixierelement vorgesehen sein, wobei dann der korrespondierende Rastvorsprung am jeweils anderen Element vorgesehen sein sollte. Günstig ist es, wenn der mindestens eine Rastvorsprung durch einen in Richtung auf die Längsachse hin weisenden Ringvorsprung am Niederhalteelement ausgebildet ist. Weist das Niederhalteelement zwei oben beschriebene, einander diametral gegenüberliegende Schlitze auf, ist somit folglich auch der Ringvorsprung in zwei sich in Umfangsrichtung erstreckende Abschnitt geteilt, die dann jeweils an den Niederhaltevorsprüngen angeordnet beziehungsweise ausgebildet.

Um das in Eingriff Bringen des mindestens einen Rastvorsprungs mit der mindestens einen Rastausnehmung zu vereinfachen, ist es vorteilhaft, wenn der mindestens eine Rastvorsprung am Niederhalteelement im Bereich der Niederhaltevorsprünge angeordnet ist. Eine solche Ausgestaltung ermöglicht es insbesondere, dass der mindestens eine Rastvorsprung sowohl in Richtung auf die Längsachse hin als auch in einer Richtung von der Längsachse weg bewegt werden kann infolge einer Bewegung der Niederhaltevorsprünge.

Günstigerweise ist die mindestens eine Rastausnehmung am Fixierelement zwischen dem Kopf und dem proximalen Außengewindeabschnitt ausgebildet. So ist es auf einfache Weise möglich, dass der mindestens eine Rastvorsprung in diese Rastausnehmung eingreift. Zudem lässt sich eine solche Rastausnehmung einfach herstellen, insbesondere durch Ausbildung eines gewindefreien Abschnitts zwischen dem Außengewinde und einem Kopf des Fixierelements.

Um eine axiale Positionierung des Niederhalteelements am Fixierelement auf einfache Weise realisieren zu können, ist es vorteilhaft, wenn in der Verbindungsstellung der mindestens eine Rastvorsprung proximalseitig am proximalen Ende des proximalen Außengewindeabschnitts anliegt oder anschlägt. Liegt insbesondere gleichzeitig ein Haltering des Niederhalteelements am Anschlag des Fixierelements an, ist das Niederhalteelement axial am Fixierelement gegen eine Relativbewegung bezogen auf das Fixierelement gesichert gelagert. Um das Fixierelement in gewünschter Weise mit dem Halteabschnitt in Eingriff bringen zu können, ist es günstig, wenn der distale Fixierelementabschnitt eine in proximaler Richtung weisend geöffnete distale Werkzeugelementaufnahme aufweist. Die distale Werkzeugelementaufnahme kann mit einem dafür vorgesehenen Werkzeug in Eingriff gebracht werden und der distale Fixierelementabschnitt mit dem Halteabschnitt derart in Eingriff gebracht werden, dass das Verbindungselement mittels des Fixierelements in definierter Weise am Befestigungselement festgelegt werden kann. Die Werkzeugelementaufnahme kann im Querschnitt vieleckig ausgebildet sein, beispielsweise kann sie einen sechseckigen Querschnitt aufweisen. Denkbar sind auch Werkzeugelementaufnahmen in Form eines Innenvielrunds oder von zwei, drei oder mehr Bohrungen, welche überlappend mit einer inneren Wandfläche des distalen Fixierelementabschnitts an diesem ausgebildet sind.

Vorzugsweise erstreckt sich die distale Werkzeugelementaufnahme im Wesentlichen über eine Länge des distalen Fixierelementabschnitts in axialer Richtung. Damit lässt sich im Wesentlichen die gesamte verfügbare Länge des distalen Fixierelementabschnitts nutzen, um mit einem Einbringwerkzeug in Eingriff gebracht zu werden.

Ferner ist es vorteilhaft, wenn der proximale Fixierelementabschnitt eine in proximaler Richtung weisend geöffnete proximale Werkzeugelementaufnahme aufweist. So kann beispielsweise das Fixierelement vor Durchtrennen der Sollbruchstelle mit einem Einbringwerkzeug, welches mit der proximalen Werkzeugelementaufnahme in Eingriff bringbar ist, in distaler Richtung bewegt und an den Halteabschnitt heran und gegebenenfalls mit diesem in Eingriff gebracht werden.

Vorzugsweise erstreckt sich die proximale Werkzeugelementaufnahme über eine Länge des Fixierelements, welche einer Länge oder im Wesentlichen einer Länge des Kopfs in axialer Richtung entspricht. Auf diese Weise kann ein sicherer Eingriff eines Einbringwerkzeugs, beispielsweise eines Schraubendrehers, mit der proximalen Werkzeugelementaufnahme sichergestellt werden. Die proximale Werkzeugelementaufnahme kann insbesondere einen mehreckigen, insbesondere vier- oder sechseckigen, Querschnitt aufweisen oder aber auch in Form eines Innenvielrund oder durch Anbringen entsprechender Bohrungen, die mit einer Innenwand des Fixierelements in diesem Bereich überlappen, ausgebildet sein.

Vorzugsweise ist ein distales Ende des Fixierelements verschlossen. Es bildet somit gleichzeitig einen distalen Anschlag für ein Einbringwerkzeug, beispielsweise ein Einschraubwerkzeug, um den distalen Fixierelementabschnitt mit dem Halteabschnitt in Eingriff zu bringen und an diesem festzulegen, beispielsweise durch Verschrauben.

Ferner kann es vorteilhaft sein, wenn das distale Ende des Fixierelements mit mindestens einer Durchbrechung versehen ist. Vorzugsweise entspricht die Zahl der Durchbrechungen der Zahl der in einer Innenwand des Fixierelements vorgesehenen Ausnehmungen zur Ausbildung eines Innenvielrund beziehungsweise der distalen Werkzeugelementaufnahme.

Zur Erhöhung einer Stabilität des Befestigungselements ist es vorteilhaft, wenn das Fixierelement einstückig ausgebildet ist. Beispielsweise kann es aus einem körperverträglichen Metall hergestellt sein, welches geeignet ist, die zur Festlegung des Verbindungselements erforderlichen Haltekräfte aufzunehmen.

Vorzugsweise ist das Niederhalteelement einstückig ausgebildet. Es lässt sich so beispielsweise einfach herstellen, insbesondere durch Spritzgießen aus einem dampfsterilisierbaren Kunststoff wie zum Beispiel Polyetheretherketon (PEEK).

Ferner kann es vorteilhaft sein, wenn das Fixierelement und das Niederhalteelement aus unterschiedlichen Materialen ausgebildet sind. Insbesondere können die beiden Elemente aus für den jeweiligen Zweck besonders geeigneten Materialien gebildet werden, beispielsweise das Niederhalteelement aus einem relativ weichen Material, welches das Verbindungselement beim Niederhalten nicht beschädigen kann, beispielsweise einem sterilisierbaren, körperverträglichen Kunststoff. Das Fixierelement kann im Gegensatz zum Niederhalteelement beispielsweise aus einem körperverträglichen Metall, insbesondere Titan oder einer Titanlegierung, hergestellt sein.

Gemäß einer weiteren bevorzugten Ausführungsform der Erfindung ist es vorteilhaft, wenn mindestens eines der chirurgischen Befestigungselemente das Wirbelsäulenstabilisierungssystems in Form einer der oben beschriebenen, vorteilhaften Ausführungsformen chirurgischer Befestigungselemente ausgebildet ist. Das Wirbelsäulenstabilisierungssystem weist dann insgesamt die oben angegebenen Vorteile auf.

Gemäß einer weiteren bevorzugten Ausführungsform des erfindungsgemäßen Wirbelsäulenstabilisierungssystems kann vorgesehen sein, dass es eine Führungshülse umfasst, welche mit dem Halteabschnitt temporär koppelbar ist und ein Innengewinde aufweist, welches zum Außengewinde des Fixierelements korrespondiert. Beispielsweise kann die Führungshülse als Teil eines vom Wirbelsäulenstabilisierungssystem umfassten Einsetzinstrumentariums ausbildet sein. Durch das Innengewinde an der Führungshülse ist es möglich, beispielsweise das Fixierelement mit der Führungshülse zu verschrauben, um so dessen Position in axialer Richtung vorzugeben.

Günstig ist es, wenn die Führungshülse einen in proximaler Richtung weisenden Anschlag für den Kopf und/oder den Haltering des Fixierelements aufweist. Auf diese Weise ist es möglich, das Fixierelement, insbesondere dessen proximalen Fixierelementabschnitt, nur bis zu dem in proximaler Richtung weisenden Anschlag an der Führungshülse zu bewegen. Das Fixierelement kann so in definierter Weise mit der Führungshülse in Eingriff gebracht und mit dieser verklemmt werden, so dass in einem nächsten Schritt insbesondere der distale Fixierelementabschnitt durch Zerstören der Sollbruchstelle vom proximalen Fixierelementabschnitt getrennt und vorzugsweise kraftfrei mit dem Innengewinde am Halteabschnitt in Eingriff gebracht werden kann, um das Verbindungselement in der Verbindungselementaufnahme festzulegen.

Um ein kraftfreies in Eingriff Bringen des distalen Fixierelementabschnitts mit dem Innengewinde des Halteabschnitts zu ermöglichen, ist es vorteilhaft, wenn in einer Montagestellung, in welcher die Führungshülse mit dem Halteabschnitt gekoppelt ist und das Fixierelement an dem in proximaler Richtung weisenden Anschlag der Führungshülse anschlägt, der distale Fixierelementabschnitt distalseitig über das Innengewinde der Führungshülse vorsteht. So kann der distale Fixierelementabschnitt vom proximalen Fixierelementabschnitt durch Zerstören der Sollbruchstelle getrennt werden und ohne Krafteinwirkung in distaler Richtung weiter bewegt und mit dem Halteabschnitt verbunden werden.

Ferner kann es günstig sein, wenn in der Montagestellung der distale Führungselementabschnitt mit dem Innengewinde am Halteabschnitt außer Eingriff steht. So kann der distale Fixierelementabschnitt vom proximalen Fixierelementabschnitt getrennt und kraftfrei mit dem Innengewinde am Halteabschnitt in Eingriff gebracht werden.

Die nachfolgende Beschreibung bevorzugter Ausführungsformen der Erfindung dient im Zusammenhang mit den Zeichnungen der näheren Erläuterung. Es zeigen:
- Figur 1:: eine schematische Gesamtansicht eines Wirbelsäulenstabilisierungssystems bei der Implantation desselben;
- Figur 2:: eine schematische Darstellung einer mit einem in einen Wirbelkörper eingebrachten Befestigungselement gekoppelten Führungshülse;
- Figur 3A:: eine Explosionsdarstellung der Führungshülse;
- Figur 3B:: eine vergrößerte Ansicht des Bereichs A in Figur 3A;
- Figur 3C:: eine Schnittansicht längs Linie 3C-3C in Figur 3A;
- Figur 4A:: eine schematische Explosionsdarstellung eines distalen Endes der Führungshülse vor dem in Eingriff Bringen mit dem Befestigungs-element;
- Figur 4B:: eine Schnittansicht längs 4B-4B in Figur 4A;
- Figur 4C:: eine Ansicht analog Figur 4B, wobei die Führungshülse mit dem Befestigungselement gekoppelt ist;
- Figur 5A:: eine Längsschnittansicht am Befestigungselement gekoppelten Führungshülse vor dem Einbringen einer Innenhülse;
- Figur 5B:: eine vergrößerte Ansicht des Bereichs B in Figur 5A;
- Figur 6A:: eine Längsschnittansicht der mit dem Befestigungselement gekoppelten Führungshülse mit eingeschobener Innenhülse;
- Figur 6B:: eine vergrößerte Ansicht des Bereichs C in Figur 6A;
- Figur 6C:: eine vergrößerte Ansicht des Bereichs D in Figur 6A;
- Figur 7A:: eine Ansicht analog Figur 6A, wobei die Innenhülse mit dem Befestigungselement gekoppelt ist;
- Figur 7B:: eine vergrößerte Ansicht des Bereichs E in Figur 7A;
- Figur 7C:: eine vergrößerte Ansicht des Bereichs F in Figur 7A;
- Figur 7D:: eine perspektivische Ansicht des Bereichs F in Figur 7A;

- Figur 8:: eine teilweise geschnittene Ansicht der Innenhülse;
- Figur 9A:: eine Explosionsdarstellung des Fixierelements und des Niederhalteelements;
- Figur 9B:: eine perspektivische Ansicht des Fixierelements und des Niederhalteelements in der Verbindungsstellung;
- Figur 9C:: eine Schnittansicht längs Linie 9C-9C in Figur 9B;
- Figur 10A:: eine schematische, teilweise geschnittene Ansicht beim Einschrauben des Fixierelements mit Niederhalteelement durch die Innenhülse;
- Figur 10B:: eine teilweise geschnittene Ansicht ähnlich Figur 10A in der Montagestellung, in welcher der Haltering des Niederhalteelements an einem in proximaler Richtung weisenden Anschlag der Innenhülse anschlägt und das Niederhalteelement das Verbindungselement in die Verbindungselementaufnahme des Halteabschnitts drückt;
- Figur 11A:: eine Schnittansicht der Anordnung in Figur 10B vor dem Durchtrennen der Sollbruchstelle;
- Figur 11B:: eine Ansicht ähnlich Figur 11A nach dem Einschrauben des distalen Fixierelementabschnitts in das Innengewinde des Halteabschnitts zum Festlegen des Verbindungselements in der Verbindungselementaufnahme;
- Figur 12:: eine Längsschnittansicht eines weiteren Ausführungsbeispiels einer Innenhülse beim Einführen eines korrespondierend ausgebildeten Fixierelements; und
- Figur 13:: eine Schnittansicht ähnlich der Anordnung in Figur 10B eines weiteren Ausführungsbeispiels eines Fixierelements mit korrespondierend ausgebildeter Innenhülse vor dem Durchtrennen der Sollbruchstelle.

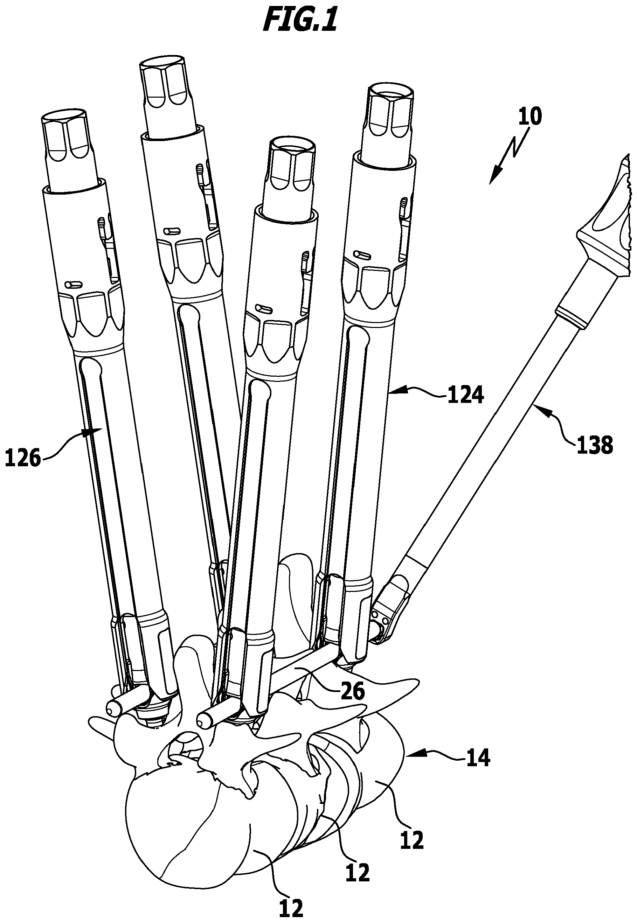

In Figur 1 ist schematisch ein insgesamt mit dem Bezugszeichen 10 bezeichnetes Wirbelsäulenstabilisierungssystem dargestellt, mit welchem zwei oder mehr zueinander benachbarte Wirbel 12 einer Wirbelsäule 14 relativ zueinander in ihrer Position stabilisiert werden können. Das Wirbelsäulenstabilisierungssystem 10 umfasst mehrere Befestigungselemente 16 in Form von Knochen- oder Pedikelschrauben 18, welche einen Befestigungsabschnitt 20 und einen Halteabschnitt 22 umfassen.

Der Halteabschnitt 22 weist eine Verbindungselementaufnahme 24 zum Aufnehmen eines Verbindungselements 26 auf und ist an einem Kopf 28 der Pedikelschraube 18 ausgebildet. Der Kopf 28 ist gabelförmig ausgebildet und weist bezogen auf eine Längsachse 30 des Befestigungselements 16 einander diametral gegenüberliegende Halteabschnitte 32 auf, welche mit einem Innengewinde 34 versehen sind. Der Befestigungsabschnitt 20 ist vorzugsweise in Form eines mit einem Knochengewinde 36 versehenen Schafts 38 ausgebildet, so dass Befestigungselement 16 in den Wirbel 12 eingeschraubt werden kann, insbesondere in einen Pedikel desselben. Alternativ kann der Befestigungsabschnitt 20 auch in Form eines Knochenhakens oder Knochennagels ausgebildet sein.

Der Befestigungsabschnitt 20 und der Halteabschnitt 22 können optional einstückig ausgebildet sein oder aber auch wie bei dem in den Figuren dargestellten Ausführungsbeispielen relativ zueinander bewegbar, beispielsweise um ein um ein Gelenkzentrum verschwenkbar, welches durch ein zwischen dem Befestigungsabschnitt 20 und dem Halteabschnitt 22 ausgebildetes Kugelgelenk definiert wird. Somit ist die Pedikelschraube 18 insbesondere in Form einer Polyaxialschraube ausgebildet.

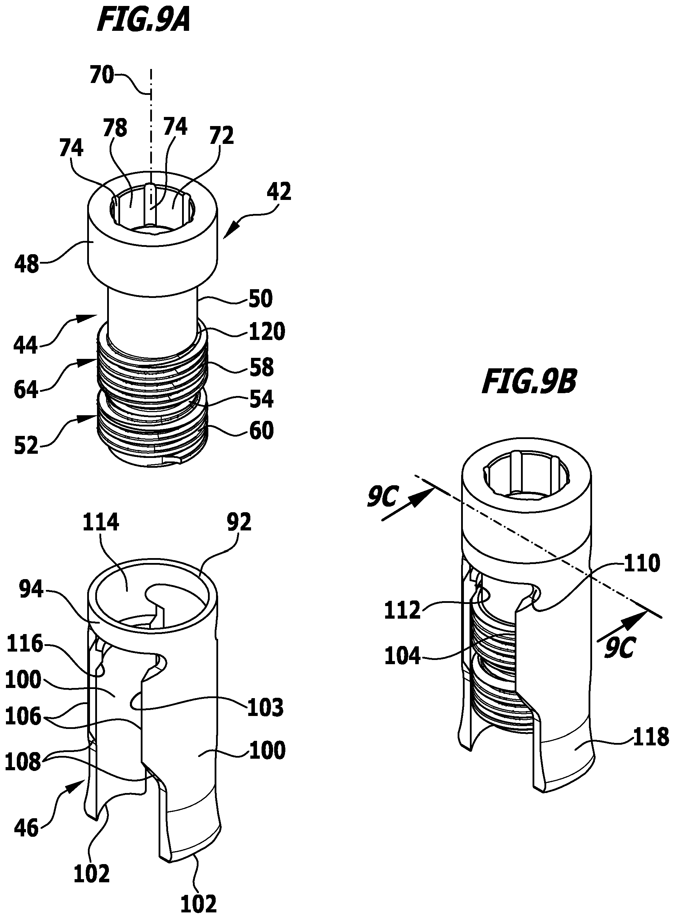

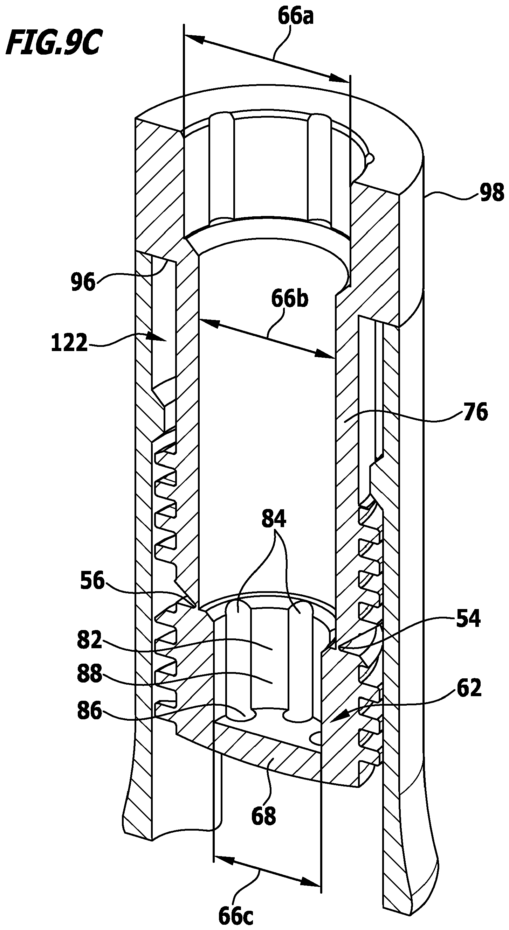

Zum Festlegen des Verbindungselements 26, welches in Form eines Verbindungsstabs 40 oder eines plattenförmigen Elements ausgebildet sein kann, dient ein Fixierelement 42, beispielsweise in Form der in Figur 9A dargestellten Fixierschraube 44. Diese ist in einer Verbindungsstellung, wie sie in Figur 9B und Figur 9C schematisch dargestellt ist, mit einem Niederhalteelement zum Drücken und/oder Niederhalten des Verbindungselements 26 verbunden beziehungsweise gekoppelt. Mit anderen Worten kann man auch sagen, dass das Fixierelement 42 das Niederhalteelement 46 trägt. Das Niederhalteelement 46 ist vorzugsweise rotierbar am Fixierelement 42 gelagert, axial jedoch unverschieblich.

Der Aufbau des Fixierelements 42 und des Niederhalteelements 46 wird nachfolgend in Verbindung mit den Figuren 9A bis 9C näher erläutert.

Die Fixierschraube 44 weist einen zylindrischen Kopf 48 auf, an den sich distalseitig ein im Durchmesser reduzierter, gewindefreier Schaftabschnitt 50 anschließt. An den Schaftabschnitt 50 schließt sich distalseitig ein Außengewinde 52 an, welches durch einen eine Sollbruchstelle 54 bildenden Einstich 56 in einen proximalen Außengewindeabschnitt 58 und einen distalen Außengewindeabschnitt 60 getrennt ist. Der Einstich 56 ist in Form einer Ringnut ausgebildet, die das Fixierelement 42 auf einer Außenseite ringförmig umgibt, vorzugsweise keilförmig ausgebildet und vom Fixierelement 42 weg weisend geöffnet ist. Das Fixierelement 42 wird durch die Sollbruchstelle 54 in einen distalen Fixierelementabschnitt 62 und einen proximalen Fixierelementabschnitt 64 unterteilt. Insgesamt ist das Fixierelement 42 einstückig ausgebildet, vorzugsweise aus einem körperverträglichen Metall, beispielsweise Titan oder einer Titanlegierung.

Das Fixierelement ist innen hohl, also insgesamt im Wesentlichen hülsenförmig ausgebildet. Ein Innendurchmesser des Fixierelements 42 verringert sich in seinem Inneren zweistufig. Im Bereich des Kopfs 48 ist ein Innendurchmesser 66a etwas größer als ein Innendurchmesser 66b im restlichen Teil des proximalen Fixierelementabschnitts 64. Ein Innendurchmesser 66c im Bereich des distalen Fixierelementabschnitts 62 ist nochmals etwas kleiner als der Innendurchmesser 66b. Der distale Fixierelementabschnitt 62 ist distalseitig mit einem Boden 68 verschlossen, welcher sich quer zu einer Längsachse 70 des Fixierelements 42 erstreckt.

Im Bereich des Kopfs 48 ist eine proximaler Richtung weisend geöffnete proximale Werkzeugelementaufnahme 72 ausgebildet, und zwar durch insgesamt sechs parallel zur Längsachse 70 ausgerichtete Bohrungen, die teilweise in eine hohlzylindrische Hülsenwand 76 des Fixierelements 42 im Bereich des Kopfs 48 eingebracht sind. So wird insgesamt ein Innenvielrund 78 ausgebildet, welcher mit einem korrespondierenden Einschraubwerkzeug 80 in Eingriff bringbar ist.

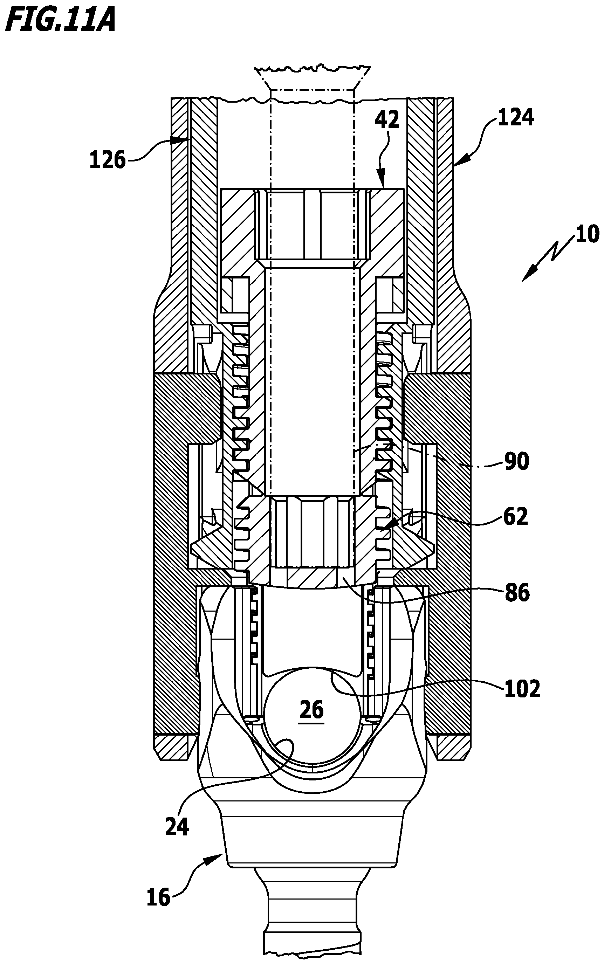

Eine im Wesentlichen analog ausgebildete distale Werkzeugelementaufnahme 82 ist im Inneren des distalen Fixierelementabschnitts 62 ausgebildet durch Einbringen von insgesamt sechs parallel zur Längsachse 70 ausgerichteten Bohrungen 84, die teilweise in die Hülsenwand 76 im Bereich des distalen Fixierelementabschnitts 62 eingreifen. Optional können die Bohrungen 84, wie in Figur 9C dargestellt, auch den Boden 74 durchstoßen und bilden somit Durchbrechungen 86 desselben. Insgesamt weist der distale Fixierelementabschnitt 62 somit ebenfalls einen Innenvielrund 88 auf, der die distale Werkzeugelementaufnahme 82 definiert. Diese kann mit einem korrespondierend ausgebildeten Einschraubwerkzeug 90, wie schematisch in Figur 11A dargestellt, in Eingriff gebracht werden.

Das Niederhalteelement 46 ist im Wesentlichen hülsenförmig ausgebildet und weist an seinem proximalen Ende 92 einen in sich geschlossenen Haltering 94 auf. Das Ende 92 liegt in der Verbindungsstellung an einer in distaler Richtung weisenden, ringförmigen Anschlagfläche 96 des einen Anschlag 98 bildenden Kopfs 48 an. Vom Haltering 94 erstrecken sich in distaler Richtung zwei, bezogen auf die Längsachse 70 einander diametral gegenüberliegende Niederhaltevorsprünge 100, deren distale Enden 102 in der Verbindungsstellung distalseitig über den Boden 68 vorstehen. Die Enden 102 sind in distaler Richtung weisend konkav gekrümmt und können optional an eine Krümmung des Elements 26 angepasst sein. Ein Außendurchmesser des Halterings 94 entspricht einem Außendurchmesser des Kopfs 48, so dass in der Verbindungsstellung eine im Wesentlichen zylindrische Gesamtanordnung aus dem Fixierelement 42 und dem Niederhalteelement 46 gebildet wird, wie dies schematisch in Figur 9B dargestellt ist.

Die Niederhaltevorsprünge 100 sind durch bezogen auf die Längsachse 70 einander diametral gegenüberliegende, Ausnehmungen 103 bildende Schlitze 104 getrennt. Seitlich sind an den Niederhaltevorsprüngen 100 in Umfangsrichtung abstehende und jeweils aufeinander zu weisende Führungsvorsprünge 106 ausgebildet, welche in distaler beziehungsweise im Wesentlichen in distaler Richtung weisende, geneigte Aufgleitflächen 108 definieren. Die Führungsvorsprünge 106 sind vom Haltering 94 etwas beabstandet, so dass in Umfangsrichtung geöffnete Einschnitte 110 ausgebildet werden.

An den Niederhaltevorsprüngen 100 sind Rastvorsprünge 112 ausgebildet, welche in Richtung auf die Längsachse 70 hin weisend von einer Innenseite 114 des Niederhalteelements 46 abstehen. Wären die Schlitze 104 nicht vorhanden, würden die beiden Rastvorsprünge 112 durch einen Ringvorsprung ausgebildet. Die Rastvorsprünge 112 sind etwas distalseitig der Einschnitte 110 an den Niederhaltevorsprüngen 100 angeordnet. In distaler Richtung weisend sind die Rastvorsprünge 112 abgeschrägt und weisen somit ebenfalls Aufgleitflächen 116 auf.

Etwas distalseitig der Führungsvorsprünge 106 nimmt ein Außendurchmesser des Niederhalteelements 46 leicht zu, so dass zum jeweiligen Ende 102 hin ein schwach konischer Außenflächenabschnitt 118 gebildet wird.

Der gewindefreie Schaftabschnitt 50 bildet für den Rastvorsprung 112 eine Rastausnehmung 120, so dass insgesamt eine Rastverbindung 122 zwischen dem Fixierelement 42 und dem Niederhalteelement 46 ausgebildet ist. In der Verbindungsstellung rastet der Rastvorsprung 112 in die Rastausnehmung 120 ein, wenn das Niederhalteelement 46 mit dem Haltering 94 voraus über das Außengewinde 52 und den Schaftabschnitt 50 geschoben wird und an den Kopf 48 anschlägt.

Das Niederhalteelement 46 ist vorzugsweise aus einem dampfsterilisierbaren Kunststoff ausgebildet, so dass die Niederhaltevorsprünge 100 beim Zusammenführen des Niederhalteelements 46 und des Fixierelements 42 etwas von der Längsachse 70 weg ausschwenken und federnd wieder in Richtung auf diese hin zurückschwenken können, wenn der Rastvorsprung 112 in die Rastausnehmung 120 eintaucht.

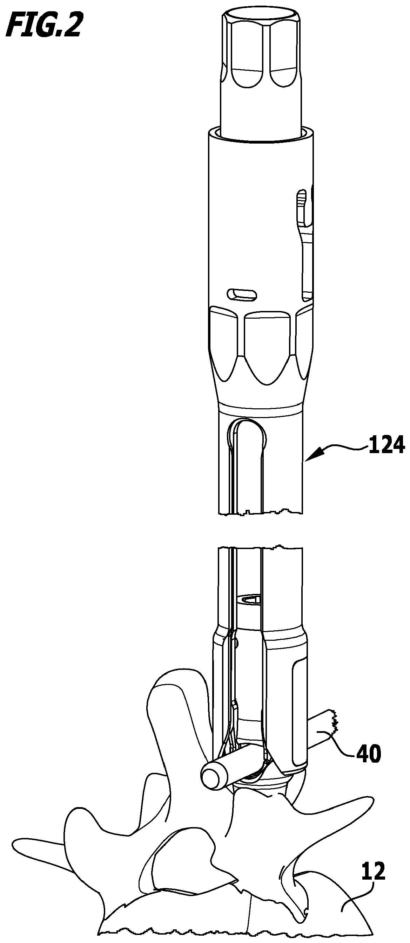

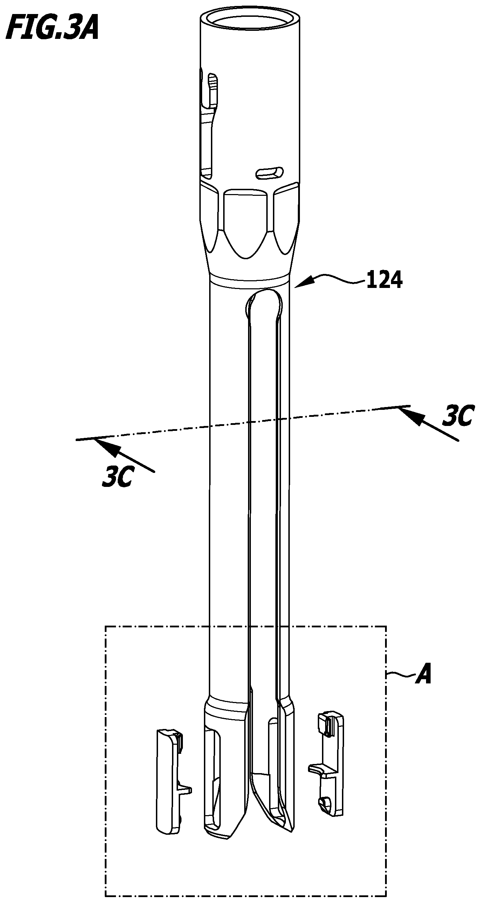

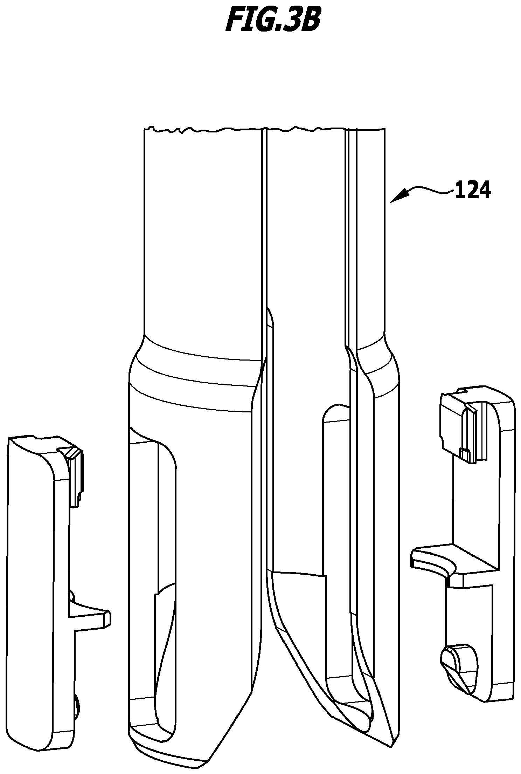

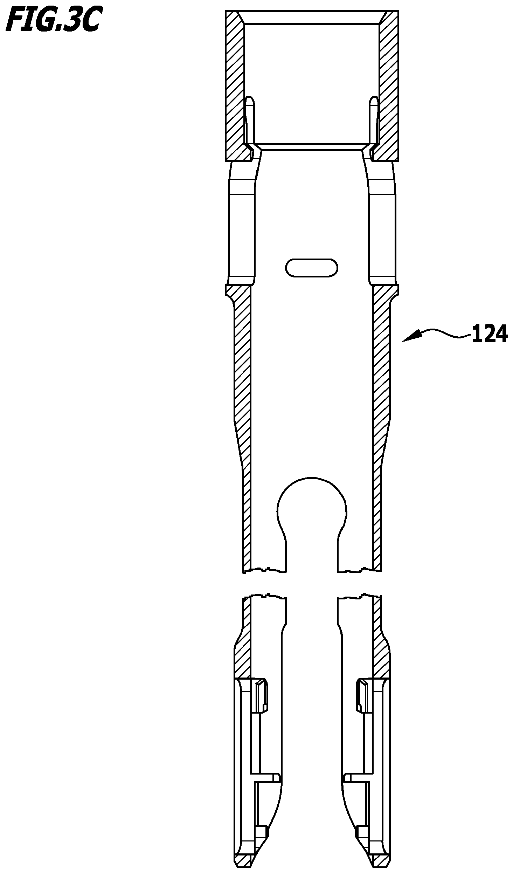

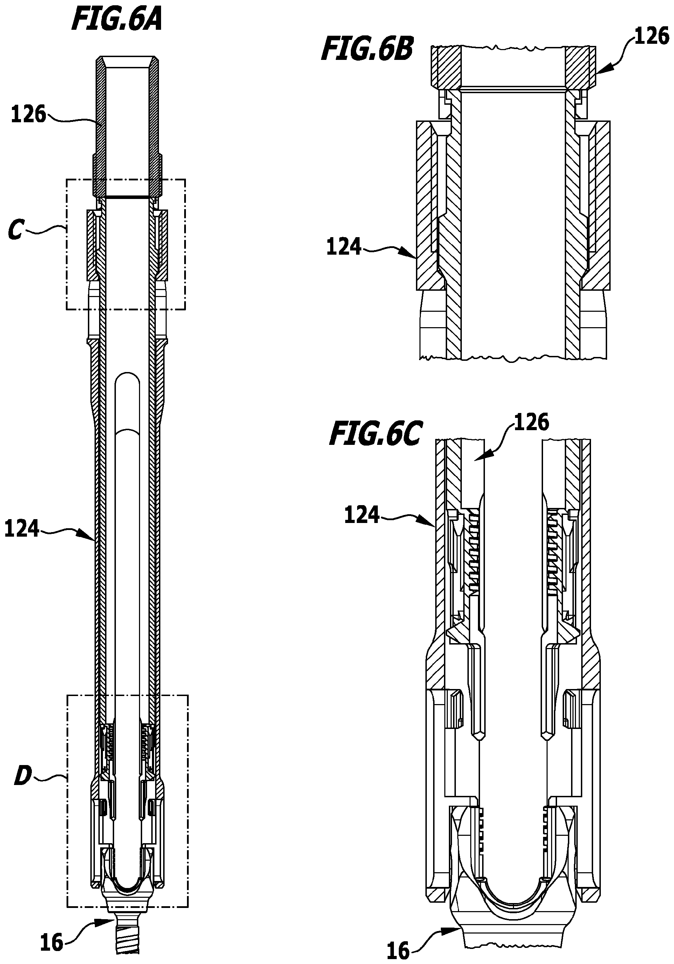

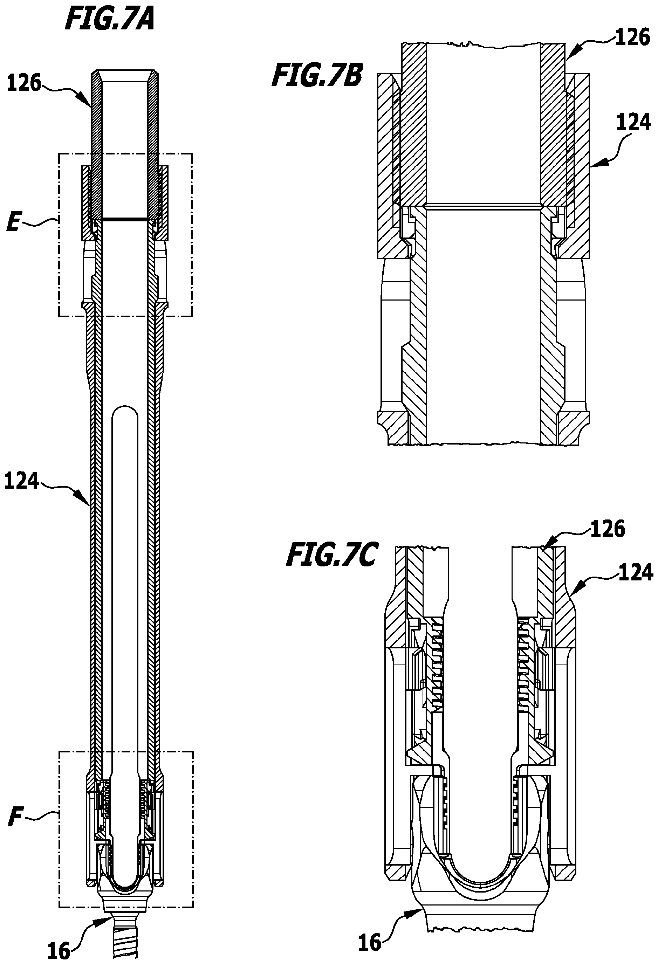

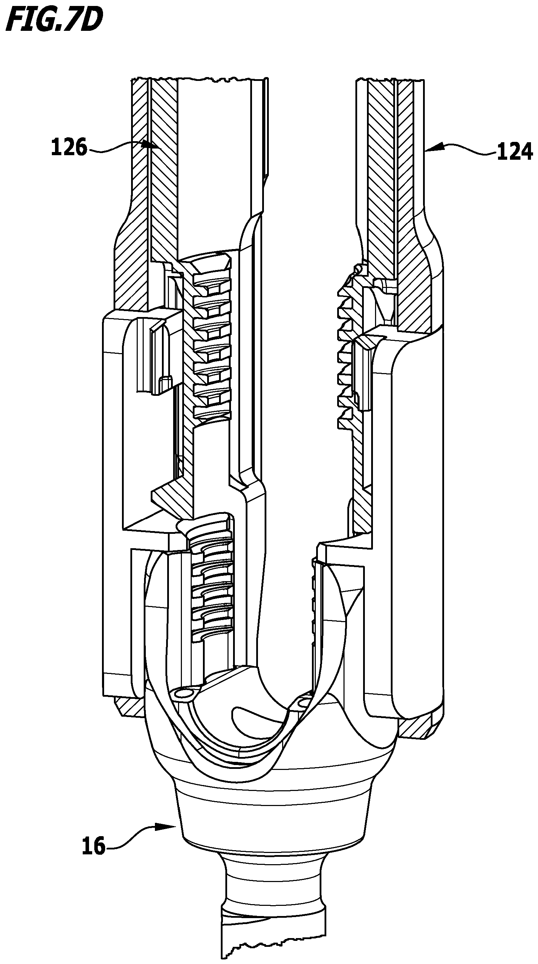

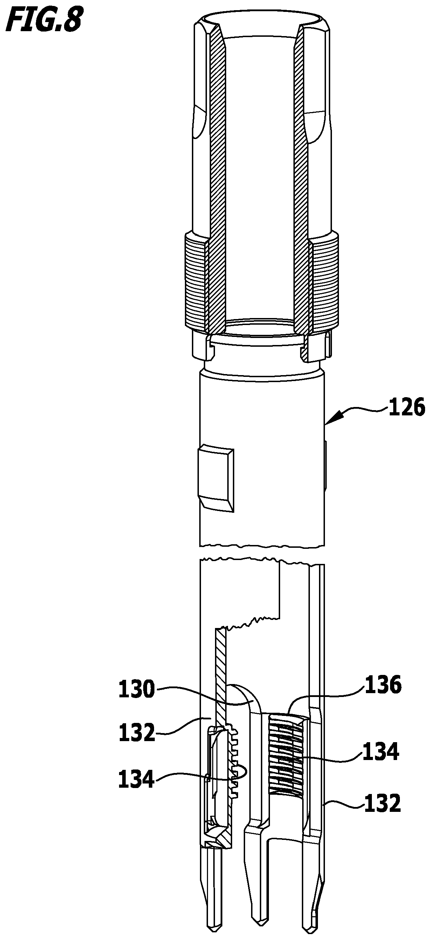

Die Implantation des Wirbelsäulenstabilisierungssystems 10 wird nachfolgend in Verbindung mit den Figuren 1 bis 11B schematisch erläutert.

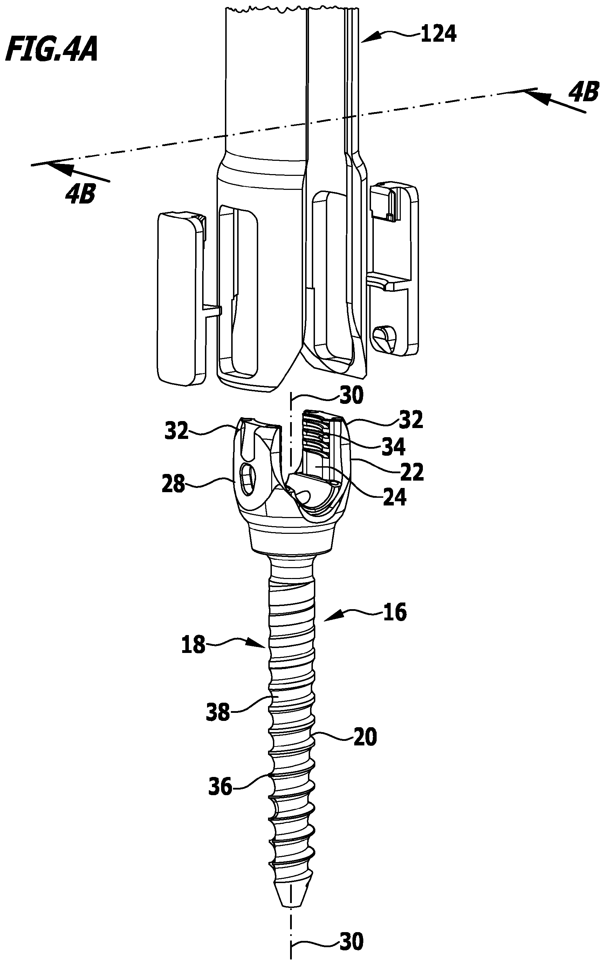

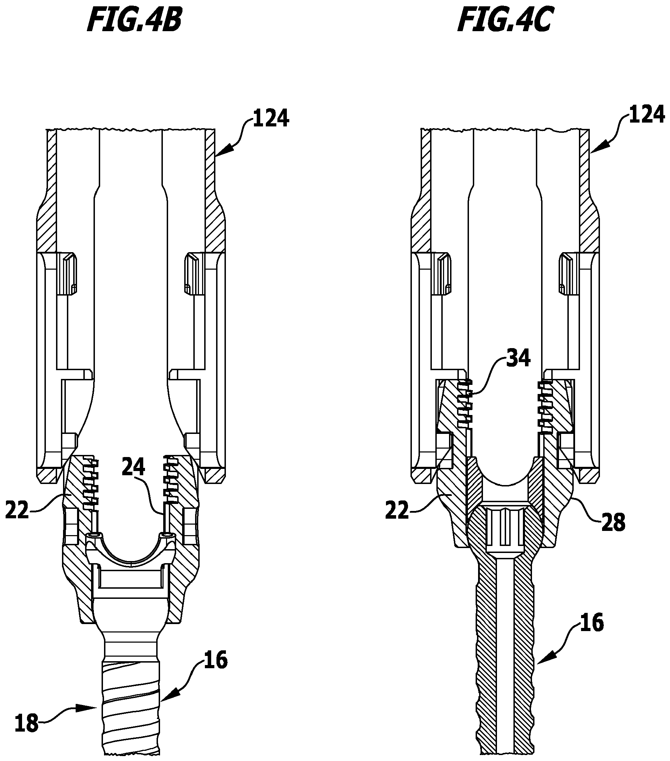

In einem ersten Schritt werden die Befestigungselemente 16 in benötigter Anzahl in die Wirbel 12 eingeschraubt. Um das Verbindungselement 26 und anschließend das Fixierelement 42 in die Halteabschnitte 22 einbringen zu können, wird in einem nächsten Schritt eine Führungshülse 124 mit dem Halteabschnitt 22 in bekannter Weise durch Verrasten gekoppelt. Dies ist schematisch in den Figuren 4B und 4C dargestellt.

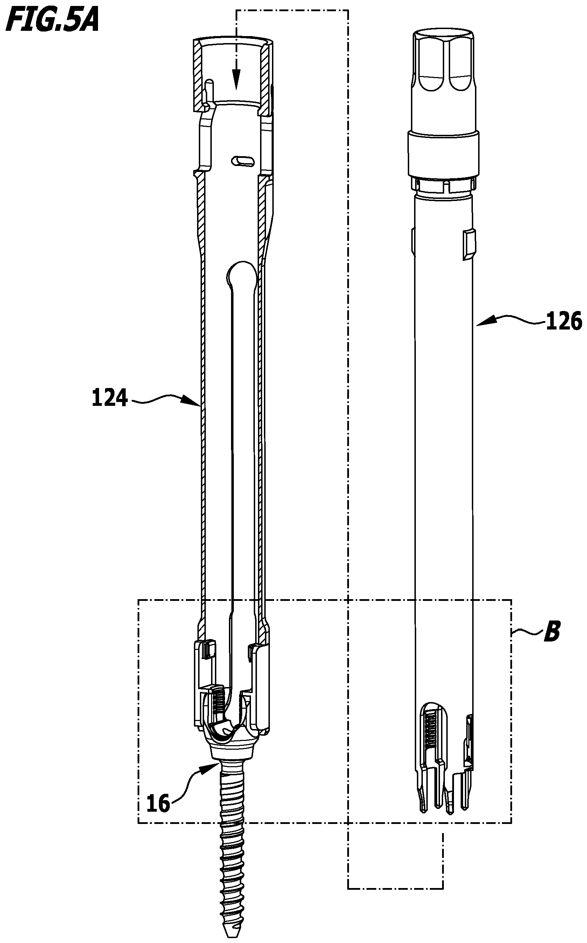

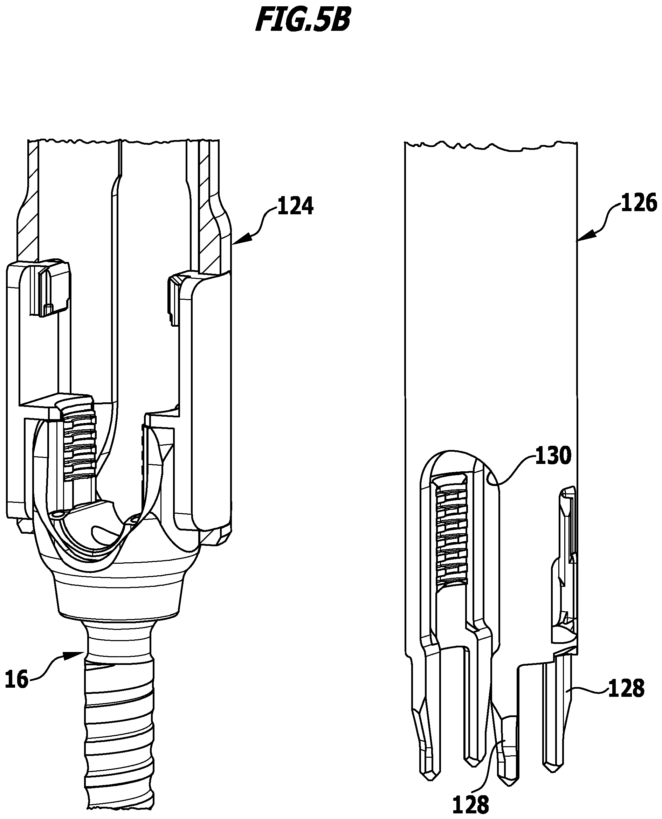

Nach Kopplung der Führungshülse 124 mit dem Befestigungselement 16 wird in einem nächsten Schritt eine zum Führen von Instrumenten dienende Innenhülse 126 von proximal her kommend in die Führungshülse 124 eingeführt. Die Innenhülse 126 weist an ihrem distalen Ende vier in distaler Richtung weisende, stabförmige Vorsprünge 128 auf, die sowohl mit dem Halteabschnitt 22 als auch der Führungshülse 124 in Eingriff bringbar sind. Das Einführen der Innenhülse 126 in die Führungshülse 124 ist in den Figuren 5A bis 7D schematisch dargestellt.

Alternativ zu den beiden vorstehend beschriebenen Schritten kann einem Operateur auch bereits eine Einheit aus Führungshülse 124 mit darin eingeschobener Innenhülse 126 von einer ihm assistierenden Person gereicht werden, die er mit dem Halteabschnitt 22 durch Verrasten koppelt

Ausgehend von ihrem distalen Ende ist die Innenhülse 124 mit zwei, bezogen auf die Längsachse 30 einander diametral gegenüberliegenden, fensterartigen Einschnitten 130 versehen. Zwischen diesen sind somit zwei ebenfalls diametral bezogen auf die Längsachse 30 einander gegenüberliegende und in distaler Richtung weisende Kopplungsvorsprünge 132 ausgebildet. Diese sind mit einem Innengewinde 134 versehen, welches gleich bemessen ist, wie das Innengewinde 34. Sowohl das Innengewinde 34 als auch das Innengewinde 134 sind korrespondierend zum Außengewinde 52 ausgebildet.

Proximalseitig schließt sich an das Innengewinde 134 eine in proximaler Richtung weisende Anschlagfläche 136 an, deren Funktion nachfolgend noch erläutert wird.

In einem nächsten Schritt kann das Verbindungselement 26 mit einem mit diesem gekoppelten Halteinstrument durch die Einschnitte 130 hindurch in die Verbindungselementaufnahmen 24 der Befestigungselemente 16 eingeführt werden, wie dies schematisch in Figur 1 dargestellt ist.

Anders als bei am Markt verfügbaren Wirbelsäulenstabilisierungssystemen 10 ist es nun nicht erforderlich, mit einem weiteren Instrument das Verbindungselement 26 in die Verbindungselementaufnahme 24 hineinzudrücken. Diese Funktion übernimmt beim Wirbelsäulenstabilisierungssystem 10 das das Niederhalteelement 46 tragende Fixierelement 42.

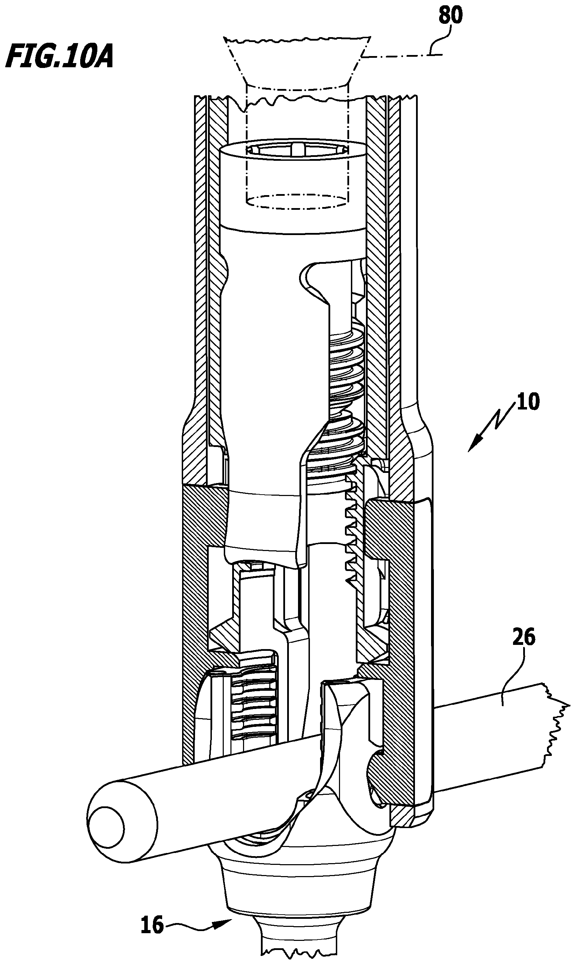

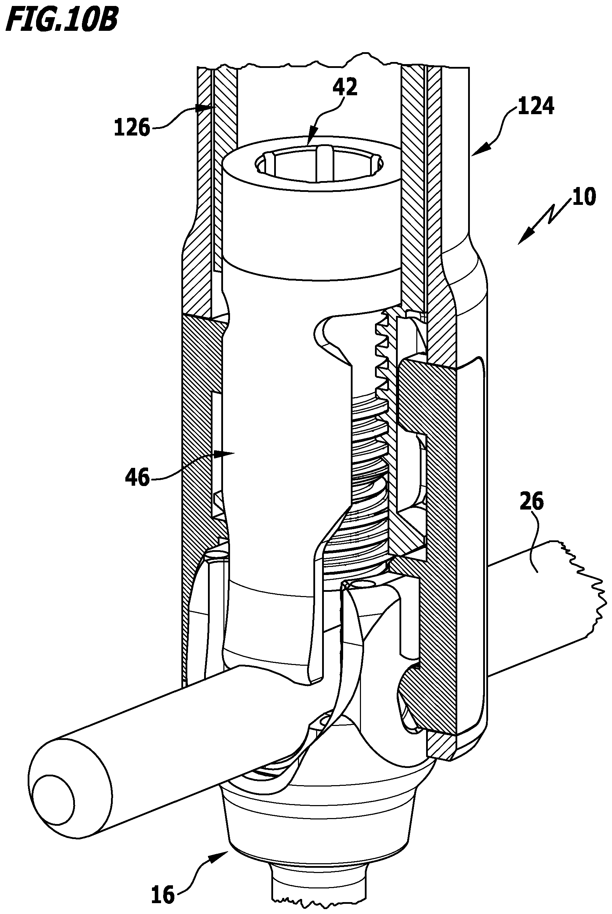

Wie in Figur 10A dargestellt, wird in einem nächsten Schritt mit dem Einschraubwerkzeug 80, welches in die proximale Werkzeugelementaufnahme 72 eingreift, das Fixierelement 42 in distaler Richtung mit dem Innengewinde 134 der Innenhülse 126 verschraubt, und zwar so weit, bis der Haltering 94 an der Anschlagfläche 136 anschlägt, wie dies schematisch in Figur 10B dargestellt ist. Beim Einschrauben treten die Enden 102 der Niederhaltevorsprünge 100 mit dem Verbindungselement 26 in Kontakt und drücken dieses beim weiteren Einschrauben des Fixierelements 42 in die Verbindungselementaufnahme 24 hinein. Das Fixierelement 42 hält mit dem Niederhalteelement 46 das Verbindungselement 26 in der Verbindungselementaufnahme 24, wie dies im Schnitt in Figur 11A nochmals aus anderer Perspektive zu erkennen ist. In dieser Figur ist auch zu erkennen, dass in dieser Montagestellung, in welcher der Haltering 94 an der Anschlagfläche 136 anschlägt, der distale Fixierelementabschnitt 62 distalseitig über das Innengewinde 134 vorsteht, also mit diesem nicht in Eingriff steht.

Um das in der Verbindungselementaufnahme 24 durch das Niederhalteelement 46 gehaltene Verbindungselement 26 endgültig am Halteabschnitt 22 zu fixieren, wird das Einschraubwerkzeug 80 zurückgezogen und das Einschraubwerkzeug 90 eingeführt und mit seinem distalen Ende mit der distalen Werkzeugelementaufnahme 82 in Eingriff gebracht. Dadurch, dass der proximale Fixierelementabschnitt 64 mit der Innenhülse 126 durch Einschrauben gekoppelt ist, kann durch Aufbringen eines entsprechenden Drehmoments die Sollbruchstelle 54 zerstört werden und der distale Fixierelementabschnitt 62 irreversibel vom proximalen Fixierelementabschnitt 58 getrennt werden. Der distale Fixierelementabschnitt 62 ist somit frei und kann unabhängig vom proximalen Fixierelementabschnitt 64 in distaler Richtung weiterbewegt und mit dem Innengewinde 34 in Eingriff gebracht und in dieses eingeschraubt werden, bis der Boden 68 an das Verbindungselement 26 anschlägt und so dass dieses schließlich klemmend in der Verbindungselementaufnahme 24 gehalten ist.

In der beschriebenen Weise werden alle benötigten Verbindungselemente 26 mit den Fixierelementen 42 an den Halteabschnitten 22 der Befestigungselemente 16 fixiert.

Abschließend wird der proximale Fixierelementabschnitt 64 mit dem daran angeordneten Niederhalteelement 46 mit dem Einschraubwerkzeug 80 in proximaler Richtung herausgeschraubt, die Innenhülse 126 in proximaler Richtung aus der Führungshülse 124 herausgezogen und die Führungshülse 124 vom Halteabschnitt 22 abgekoppelt. Alternativ kann auch die Einheit aus Führungshülse 124 und Innenhülse 126 insgesamt in einem Schritt entfernt werden. Das Wirbelsäulenstabilisierungssystem 10 ist nun an den Wirbeln 12 wie gewünscht fixiert.

Das beschriebene Wirbelsäulenstabilisierungssystem 10 kommt somit insbesondere ohne in proximaler Richtung vom Halteabschnitt 22 abstehende Verlängerungen aus, wie dies bei bekannten Systemen vorgesehen ist, wobei die Verlängerungen nach Festlegen des Fixierelements 42 am Halteabschnitt 22 entfernt werden müssen, beispielsweise durch Abrechen. Außerdem wird beim beschriebenen Wirbelsäulenstabilisierungssystem 10 das Vorsehen eines Stabeindrückinstruments, beispielsweise in Form einer weiteren, die Führungshülse 124 außen umgreifenden Niederdrückhülse, überflüssig. Diese hätte zudem den Nachteil, dass ein Hautschnitt für einen minimalinvasiven Zugang zum Implantieren des Wirbelsäulenstabilisierungssystems 10 noch etwas vergrößert werden müsste. Dieser Nachteil wird durch das vorgeschlagene Wirbelsäulenstabilisierungssystem 10 vermieden.

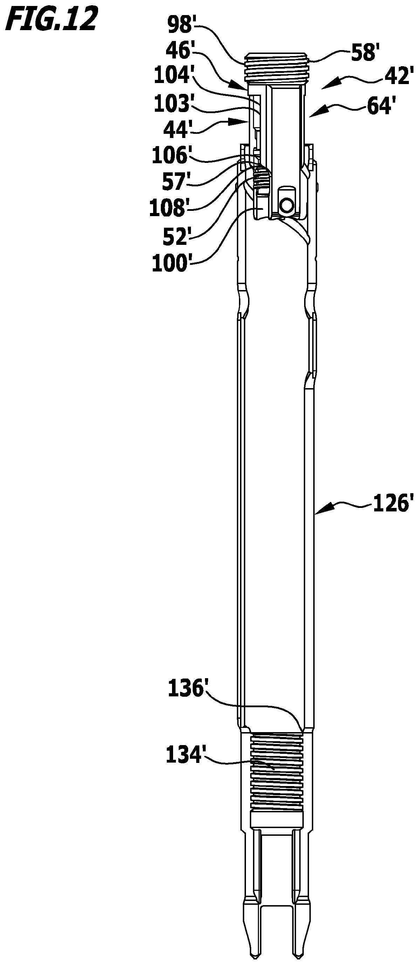

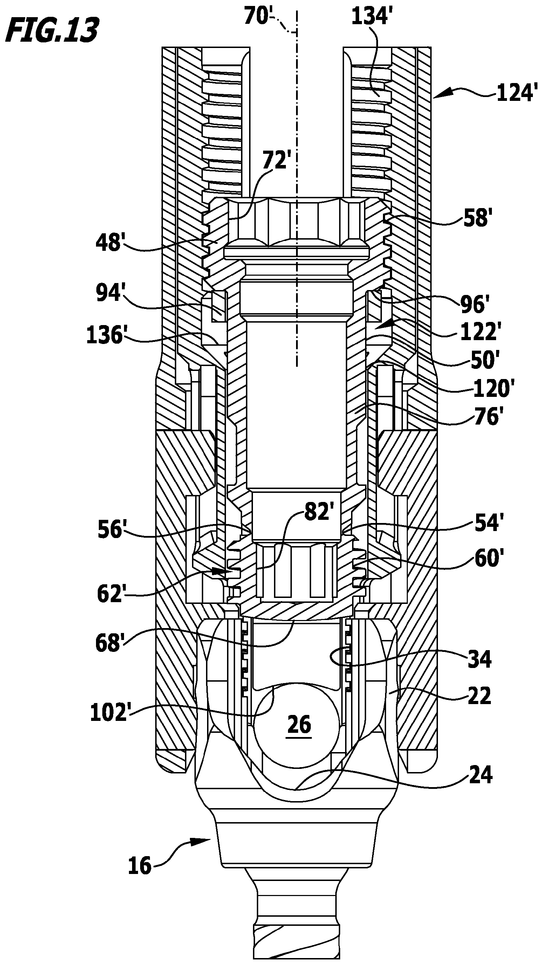

In den Figuren 12 und 13 sind alternative Ausführungsbeispiele einer Innenhülse 126' sowie eines mit dieser zusammenwirkenden Fixierelements 42' schematisch dargestellt. Sie entsprechen in ihrem Aufbau und in ihrer Funktionsweise im Wesentlichen der Innenhülse 126 sowie dem Fixierelement 42.

Der Unterschied zwischen der Innenhülse 126' und der Innenhülse 126 besteht im Wesentlichen darin, dass das Innengewinde 134' etwas weiter proximal an der Innenhülse 126' angeordnet ist als das Innengewinde 134 an der Innenhülse 126. Das Innengewinde 134' ist somit in dem Bereich angeordnet, in welchem der Innendurchmesser der Innenhülse 126' etwas erweitert ist. Das Innengewinde 134' ist also dort angeordnet, wo bei der Innenhülse 126 der im Außendurchmesser gegenüber dem Außengewinde 52 vergrößerte Kopf angeordnet ist, wenn das Fixierelement 42 maximal weit in distaler Richtung vorgeschoben ist, wie das schematisch in Figur 11A dargestellt ist. Im Gegensatz hierzu ist nun das Innengewinde 134' genau dort angeordnet, wo der Kopf 48' sitzt. Es wirkt mit dem Außengewinde des proximalen Außengewindeabschnitts 58 zusammen, welches am Kopf 48' des Fixierelements 42' ausgebildet ist. Das Fixierelement 42' weist daher zwei räumlich deutlich voneinander getrennte Außengewindeabschnitte auf, nämlich den distalen Außengewindeabschnitt 60' am distalen Fixierelementabschnitt 62' sowie den proximalen Außengewindeabschnitt 58' am proximalen Fixierelementabschnitt 64. Damit unterscheiden sich auch die proximalen und distalen Außengewindeabschnitte 58' und 60' in ihrem Durchmesser, der distale Außengewindeabschnitt 60' ist etwas kleiner im Außendurchmesser ausgebildet.

Die Innenhülse 126' weist gegenüber der Innenhülse 126 ferner einen weiteren Unterschied auf. Ausgehend von ihrem proximalen Ende ist an der Innenhülse 126' in einer inneren Wandfläche 140' derselben eine helixförmige Nut 142' ausgebildet. In diese greift ein korrespondierender Vorsprung eines Niederhaltevorsprungs 100' des Fixierelements 42' ein, so dass beim Einführen des Fixierelements 42' in die Innenhülse 126' die Niederhaltevorsprünge 100' so zu den schlitzförmigen Einschnitten 130' ausgerichtet werden, dass sie in diese eingreifen können.

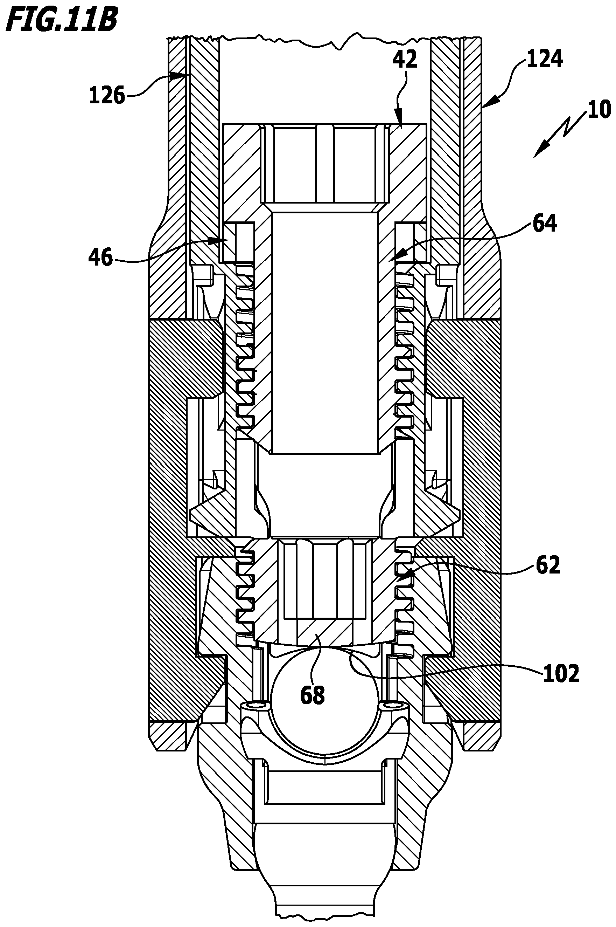

Die Funktionsweise des Fixierelements 42' entspricht im Wesentlichen der Funktion des Fixierelements 42. Es wird analog wie im Zusammenhang mit den Figuren 10A, 10B, 11A und 11B beschrieben, mit dem Einschraubwerkzeug 80, welches an die proximale Fixierelementaufnahme 72' eingreift, in distaler Richtung mit dem Innengewinde 134' der Innenhülse 126' verschraubt, und zwar so weit, bis die distalen Enden 102' der Niederhaltevorsprünge 100' am Verbindungselement 26 anschlagen. Beim weiteren Einschrauben des Fixierelements 42' drücken die Enden 102' der Niederhaltevorsprünge 100' das Verbindungselement 26 in die Verbindungselementaufnahme 24 hinein. Das Fixierelement 42' hält mit dem Niederhalteelement 46' das Verbindungselement 26 in der Verbindungelementaufnahme 24, wie dies schematisch in Figur 13 dargestellt ist. In dieser Figur ist auch gut zu erkennen, dass in dieser Montagestellung, in welcher die distalen Enden 102' der Niederhaltevorsprünge 100' am Verbindungselement 26 anschlagen, der distale Fixierelementabschnitt 62' noch nicht mit dem Innengewinde 34 in Eingriff steht.

Um das in der Verbindungselementaufnahme 24 durch das Niederhalteelement 46- gehaltene Verbindungselement 26 endgültig am Halteabschnitt 22 zu fixieren, wird das Einschraubwerkzeug 80 zurückgezogen und das Einschraubwerkzeug 90 eingeführt und mit seinem distalen Ende mit der distalen Werkzeugelementaufnahme 82' in Eingriff gebracht. Dadurch, dass der proximale Fixierelementabschnitt 64' mit der innenhülse 126' durch Einschrauben gekoppelt ist, kann durch Aufbringen eines entsprechenden Drehmoments die Sollbruchstelle 54' zerstört werden und der distale Fixierelementabschnitt 62' irreversibel vom proximalen Fixierelementabschnitt 58 getrennt werden. Der distale Fixierelementabschnitt 62' ist somit frei und kann unabhängig vom proximalen Fixierelementabschnitt 64' in distaler Richtung weiterbewegt und mit dem Innengewinde 34 in Eingriff gebracht und in dieses eingeschraubt werden, bis der Boden 68' an das Verbindungselement 26 anschlägt und so dieses schließlich klemmend in der Verbindungselementaufnahme 24 gehalten ist.

Die Funktionsweise des Wirbelsäulenstabilisierungssystems 10 mit der Innenhülse 126' und dem Fixierelement 42' ist ansonsten analog wie oben beschrieben.

Optional können die Funktionen des Einschraubwerkzeugs 80 und des Einschraubwerkzeugs 90 auch in einem einzigen Instrument vereint werden, welches insbesondere mehrteilig ausgebildet sein kann.

## Patentansprüche

1. Chirurgisches Befestigungselement (16) für ein mindestens zwei Befestigungselemente (16) und mindestens ein Verbindungselement (26) umfassendes Wirbelsäulenstabilisierungssystem (10), welches chirurgische Befestigungselement (16) einen Befestigungsabschnitt (20), einen Halteabschnitt (22) mit einer Verbindungselementaufnahme (24) und ein am Halteabschnitt (22) festlegbares Fixierelement (42; 42') zum Festlegen des Verbindungselements (26) in der Verbindungselementaufnahme (24) umfasst, wobei das Fixierelement (42; 42') ein Niederhalteelement (46; 46') zum Niederhalten des Verbindungselements (26) in die Verbindungselementaufnahme (24) trägt, **dadurch gekennzeichnet, dass** das Fixierelement (42; 42') eine Sollbruchstelle (54; 54') aufweist, welche einen distalen Fixierelementabschnitt (62; 62') und einen proximalen Fixierelementabschnitt (64; 64') trennt, und dass das Niederhalteelement (46; 46') am proximalen Fixierelementabschnitt (64; 64') angeordnet ist.

2. Chirurgisches Befestigungselement nach Anspruch 1, **dadurch gekennzeichnet, dass** das Fixierelement (42; 42') hülsenförmig oder im Wesentlichen hülsenförmig ausgebildet ist und eine eine Längsachse (70; 70') des Fixierelements (42; 42') konzentrisch umgebende Fixierelementwand (76) umfasst und/oder dass das Fixierelement (42; 42') einen in distaler Richtung wirkenden Anschlag (98; 98') für das Niederhalteelement (46; 46') umfasst.

3. Chirurgisches Befestigungselement nach Anspruch 1 oder 2, **dadurch gekennzeichnet, dass** das Fixierelement (42; 42') in Form einer Fixierschraube (44; 44') ausgebildet ist, welche ein Außengewinde (52; 52') aufweist, welches sich ausgehend von einem distalen Ende der Fixierschraube (44; 44') in proximaler Richtung erstreckt, und dass das Außengewinde (52; 52') korrespondierend zu einem am Halteabschnitt (22) ausgebildeten Innengewinde (34) ausgebildet ist.

4. Chirurgisches Befestigungselement nach Anspruch 2 oder 3, **dadurch gekennzeichnet, dass** das Fixierelement (42; 42') einen Kopf (48; 48') aufweist, welcher den Anschlag (98; 98') bildet oder umfasst.

5. Chirurgisches Befestigungselement nach einem der voranstehenden Ansprüche, **dadurch gekennzeichnet, dass** das Niederhalteelement (46; 46') am Fixierelement (42; 42') rotierbar gelagert ist und/oder dass das Niederhalteelement (46; 46') am Fixierelement (42; 42') in axialer Richtung unbeweglich oder im Wesentlichen unbeweglich gehalten ist und/ oder dass das Niederhalteelement (46; 46') hülsenförmig oder im Wesentlichen hülsenförmig ausgebildet ist und/oder dass das Niederhalteelement (46; 46') bezogen auf eine Längsachse (70; 70') zwei einander diametral gegenüberliegende Ausnehmungen (103; 103') aufweist.

6. Chirurgisches Befestigungselement nach einem der voranstehenden Ansprüche, **dadurch gekennzeichnet, dass** das Niederhalteelement (46; 46') einen Haltering (94; 94') umfasst, welcher zwei in distaler Richtung abstehende, einander diametral gegenüberliegende Niederhaltevorsprünge (100; 100') trägt.

7. Chirurgisches Befestigungselement nach Anspruch 6, **dadurch gekennzeichnet, dass** der Haltering (94; 94') ein proximales Ende des Niederhalteelements (46; 46') bildet und/oder dass die Niederhaltevorsprünge (100; 100') durch die zwei einander diametral gegenüberliegenden Ausnehmungen (103; 103') voneinander getrennt sind und/oder dass distale Enden (102; 102') der Niederhaltevorsprünge (100; 100') in distaler Richtung weisend konkav gekrümmt sind und/oder dass die Niederhaltevorsprünge (100; 100') in Umfangsrichtung weisende, seitlich abstehende Führungsvorsprünge (106; 106') tragen.

8. Chirurgisches Befestigungselement nach Anspruch 6 oder 7, **dadurch gekennzeichnet, dass** in einer Verbindungsstellung, in welcher das Niederhalteelement (46; 46') mit dem Fixierelement (42; 42') gekoppelt ist, ein proximales Ende des Halterings (94; 94') am Anschlag (98; 98') anliegt und/oder dass ein Außendurchmesser des Halterings (94; 94') einem Außendurchmesser des Kopfs (48; 48') entspricht oder im Wesentlichen entspricht.

9. Chirurgisches Befestigungselement nach einem der voranstehenden Ansprüche, **gekennzeichnet durch** eine Rastverbindung (122; 122') zum lösbaren Verbinden des Fixierelements (42; 42') und des Niederhalteelements (46; 46') in einer Verbindungsstellung.

10. Chirurgisches Befestigungselement nach einem der Ansprüche 3 bis 9, **dadurch gekennzeichnet, dass** der distale Fixierelementabschnitt (62; 62') eine in proximaler Richtung weisend geöffnete distale Werkzeugelementaufnahme (82; 82') aufweist.

11. Chirurgisches Befestigungselement nach einem der Ansprüche 3 bis 10, **dadurch gekennzeichnet, dass** der proximale Fixierelementabschnitt (64; 64') eine in proximaler Richtung weisend geöffnete proximale Werkzeugelementaufnahme (72; 72') aufweist.

12. Chirurgisches Befestigungselement nach einem der voranstehenden Ansprüche, **dadurch gekennzeichnet, dass** ein distales Ende des Fixierelements (42; 42') verschlossen ist.

13. Chirurgisches Befestigungselement nach einem der voranstehenden Ansprüche, **dadurch gekennzeichnet, dass** das Fixierelement (42; 42') einstückig ausgebildet ist und/oder dass das Niederhalteelement (46; 46') einstückig ausgebildet ist und/oder dass das Fixierelement (42; 42') und das Niederhalteelement (46; 46') aus unterschiedlichen Materialen ausgebildet sind.

14. Wirbelsäulenstabilisierungssystem (10) umfassend mindestens zwei chirurgische Befestigungselemente (16) und mindestens ein Verbindungselement (26), **dadurch gekennzeichnet, dass** mindestens eines der mindestens zwei chirurgischen Befestigungselemente (16) in Form eines Befestigungselements (16) nach einem der voranstehenden Ansprüche ausgebildet ist.

15. Wirbelsäulenstabilisierungssystem nach Anspruch 14, **dadurch gekennzeichnet, dass** es eine Führungshülse (126; 126') umfasst, welche mit dem Halteabschnitt (22) temporär koppelbar ist und ein Innengewinde (134; 134') aufweist, welches zum Außengewinde (52; 52') des Fixierelements (42; 42') korrespondiert.

## Claims

1. Surgical fastening element (16) for a spinal column stabilization system (10) comprising at least two fastening elements (16) and at least one connecting element (26), said surgical fastening element (16) comprising a fastening section (20), a holding section (22) with a connecting element receptacle (24), and a fixing element (42; 42') fixable on the holding section (22) for fixing the connecting element (26) in the connecting element receptacle (24), wherein the fixing element (42; 42') carries a holding down element (46; 46') for holding the connecting element (26) down in the connecting element receptacle (24), **characterized in that** the fixing element (42; 42') has a predetermined break-off point (54; 54') which separates a distal fixing element section (62; 62') and a proximal fixing element section (64; 64'), and **in that** the holding down element (46; 46') is arranged on the proximal fixing element section (64; 64').

2. Surgical fastening element in accordance with claim 1, **characterized in that** the fixing element (42; 42') is of sleeve-shaped or substantially sleeve-shaped construction and has a fixing element wall (76) concentrically surrounding a longitudinal axis (70; 70') of the fixing element (42; 42') and/or **in that** the fixing element (42; 42') comprises a stop (98; 98') acting in the distal direction for the holding down element (46; 46').

3. Surgical fastening element in accordance with claim 1 or 2, **characterized in that** the fixing element (42; 42') is constructed in the form of a fixing screw (44; 44'), which comprises an external thread (52; 52') starting from a distal end of the fixing screw (44; 44') and extending in the proximal direction, and **in that** the external thread (52; 52') is constructed so as to correspond to an internal thread (34) formed on the holding section (22).

4. Surgical fastening element in accordance with claim 2 or 3, **characterized in that** the fixing element (42; 42') comprises a head (48; 48') which forms or comprises the stop (98; 98').

5. Surgical fastening element in accordance with any one of the preceding claims, **characterized in that** the holding down element (46; 46') is rotatably mounted on the fixing element (42; 42') and/or **in that** the holding down element (46; 46') is held on the fixing element (42; 42') so as to be immovable or substantially immovable in the axial direction and/or **in that** the holding down element (46; 46') is of sleeve-shaped or substantially sleeve-shaped construction and/or **in that** the holding down element (46; 46') comprises, in relation to a longitudinal axis (70; 70'), two diametrically opposed recesses (103; 103').

6. Surgical fastening element in accordance with any one of the preceding claims, **characterized in that** the holding down element (46; 46') comprises a holding ring (94; 94') which carries two diametrically opposed holding down projections (100; 100') protruding in the distal direction.

7. Surgical fastening element in accordance with claim 6, **characterized in that** the holding ring (94; 94') forms a proximal end of the holding down element (46; 46') and/or **in that** the holding down projections (100; 100') are separated from each other by the two diametrically opposed recesses (103; 103') and/or **in that** distal ends (102; 102') of the holding down projections (100; 100') are concavely curved facing in the distal direction and/or **in that** the holding down projections (100; 100') carry guide projections (106; 106') facing in the circumferential direction and protruding at the side.

8. Surgical fastening element in accordance with claim 6 or 7, **characterized in that** in a connected position in which the holding down element (46; 46') is coupled to the fixing element (42; 42'), a proximal end of the holding ring (94; 94') lies against the stop (98; 98') and/or **in that** an outer diameter of the holding ring (94; 94') corresponds or corresponds substantially to an outer diameter of the head (48; 48').

9. Surgical fastening element in accordance with any one of the preceding claims, **characterized by** a locking connection (122; 122') for releasably connecting the fixing element (42; 42') and the holding down element (46; 46') in a connected position.

10. Surgical fastening element in accordance with any one of claims 3 to 9, **characterized in that** the distal fixing element section (62; 62') comprises a distal tool element receptacle (82; 82') which is open facing in the proximal direction.

11. Surgical fastening element in accordance with any one of claims 3 to 10, **characterized in that** the proximal fixing element section (64; 64') comprises a proximal tool element receptacle (72; 72') which is open facing in the proximal direction.

12. Surgical fastening element in accordance with any one of the preceding claims, **characterized in that** a distal end of the fixing element (42; 42') is closed.

13. Surgical fastening element in accordance with any one of the preceding claims, **characterized in that** the fixing element (42; 42') is of integral construction and/or **in that** the holding down element (46; 46') is of integral construction and/or **in that** the fixing element (42; 42') and the holding down element (46; 46') are formed from different materials.

14. Spinal column stabilization system (10) comprising at least two surgical fastening elements (16) and at least one connecting element (26), **characterized in that** at least one of the at least two surgical fastening elements (16) is constructed in the form of a surgical fastening element (16) according to any one of the preceding claims.

15. Spinal column stabilization system in accordance with claim 14, **characterized in that** it comprises a guide sleeve (126; 126'), which is adapted to be temporarily coupled to the holding section (22) and has an internal thread (134; 134') which corresponds to the external thread (52; 52') of the fixing element (42; 42').

## Revendications

1. Elément d'assujettissement chirurgical (16) pour un système de stabilisation de la colonne vertébrale (10) comprenant au moins deux éléments d'assujettissement (16) et au moins un élément de liaison (26), lequel élément d'assujettissement chirurgical (16) comprend un segment d'assujettissement (20), un segment de maintien (22) avec un logement d'élément de liaison (24) et un élément de fixation (42 ; 42') qui peut être fixé au segment de maintien (22) pour la fixation de l'élément de liaison (26) dans le logement d'élément de liaison (24), où l'élément de fixation (42 ; 42') porte un élément de retenue (46 ; 46') pour la retenue de l'élément de liaison (26) dans le logement d'élément de liaison (24), **caractérisé en ce que** l'élément de fixation (42 ; 42') présente une zone destinée à la rupture (54 ; 54') qui sépare un segment d'élément de fixation distal (62 ; 62') et un segment d'élément de fixation proximal (64 ; 64'), et **en ce que** l'élément de retenue (46 ; 46') est disposé sur le segment d'élément de fixation proximal (64 ; 64').

2. Elément d'assujettissement chirurgical selon la revendication 1, **caractérisé en ce que** l'élément de fixation (42 ; 42') est agencé sous forme de douille ou sensiblement sous forme de douille et comprend une paroi d'élément de fixation (76) entourant un axe longitudinal (70 ; 70') de l'élément de fixation (42 ; 42') de manière concentrique et/ou **en ce que** l'élément de fixation (42 ; 42') comprend une butée (98 ; 98') agissant dans la direction distale pour l'élément de retenue (46 ; 46').

3. Elément d'assujettissement chirurgical selon la revendication 1 ou 2, **caractérisé en ce que** l'élément de fixation (42 ; 42') est agencé sous forme d'une vis de fixation (44 ; 44') qui présente un filet externe (52 ; 52') qui s'étend dans la direction proximale à partir d'une extrémité distale de la vis de fixation (44 ; 44'), et **en ce que** le filet externe (52 ; 52') est agencé d'une manière correspondant à un filet interne (34) agencé sur le segment de maintien (22).

4. Elément d'assujettissement chirurgical selon la revendication 2 ou 3, **caractérisé en ce que** l'élément de fixation (42 ; 42') présente une tête (48 ; 48') qui forme ou comprend la butée (98 ; 98').

5. Elément d'assujettissement chirurgical selon l'une des revendications précédentes, **caractérisé en ce que** l'élément de retenue (46 ; 46') est monté à rotation sur l'élément de fixation (42 ; 42') et/ou **en ce que** l'élément de retenue (46 ; 46') est maintenu sur l'élément de fixation (42 ; 42') de manière immobile ou sensiblement immobile en direction axiale et/ou **en ce que** l'élément de retenue (46 ; 46') est agencé sous forme de douille ou sensiblement sous forme de douille et/ou **en ce que** l'élément de retenue (46 ; 46') présente deux évidements (103 ; 103') diamétralement opposés l'un à l'autre par rapport à un axe longitudinal (70 ; 70').

6. Elément d'assujettissement chirurgical selon l'une des revendications précédentes, **caractérisé en ce que** l'élément de retenue (46 ; 46') comprend une bague de maintien (94 ; 94') qui porte deux protubérances de retenue (100 ; 100') diamétralement opposées l'une à l'autre, qui s'étendent dans la direction distale.

7. Elément d'assujettissement chirurgical selon la revendication 6, **caractérisé en ce que** la bague de maintien (94 ; 94') forme une extrémité proximale de l'élément de retenue (46 ; 46') et/ou **en ce que** les protubérances de retenue (100; 100') sont séparées l'une de l'autre par les deux évidements (103 ; 103') diamétralement opposés l'un à l'autre et/ou **en ce que** les extrémités distales (102 ; 102') des protubérances de retenue (100 ; 100') sont courbées de manière concave dans la direction distale et/ou **en ce que** les protubérances de retenue (100 ; 100') portent des protubérances de guidage (106 ; 106') qui s'étendent latéralement dans la direction de la périphérie.

8. Elément d'assujettissement chirurgical selon la revendication 6 ou 7, **caractérisé en ce que** dans une position de liaison dans laquelle l'élément de retenue (46 ; 46') est couplé avec l'élément de fixation (42 ; 42'), une extrémité proximale de la bague de maintien (94 ; 94') repose sur la butée (98 ; 98') et/ou **en ce qu'**un diamètre externe de la bague de maintien (94 ; 94') correspond ou correspond sensiblement à un diamètre externe de la tête (48 ; 48').

9. Elément d'assujettissement chirurgical selon l'une des revendications précédentes, **caractérisé par** une liaison à encliquetage (122 ; 122') pour la liaison amovible de l'élément de fixation (42 ; 42') et de l'élément de retenue (46 ; 46') dans une position de liaison.

10. Elément d'assujettissement chirurgical selon l'une des revendications 3 à 9, **caractérisé en ce que** le segment d'élément de fixation distal (62 ; 62') présente un logement d'élément d'outil distal (82 ; 82') ouvert dans la direction proximale.

11. Elément d'assujettissement chirurgical selon l'une des revendications 3 à 10, **caractérisé en ce que** le segment d'élément de fixation proximal (64 ; 64') présente un logement d'élément d'outil proximal (72 ; 72') ouvert dans la direction proximale.

12. Elément d'assujettissement chirurgical selon l'une des revendications précédentes, **caractérisé en ce qu'**une extrémité distale de l'élément de fixation (42 ; 42') est fermée.

13. Elément d'assujettissement chirurgical selon l'une des revendications précédentes, **caractérisé en ce que** l'élément de fixation (42 ; 42') est agencé d'une pièce et/ou **en ce que** l'élément de retenue (46 ; 46') est agencé d'une pièce et/ou **en ce que** l'élément de fixation (42 ; 42') et l'élément de retenue (46 ; 46') sont agencés en matériaux différents.

14. Système de stabilisation de la colonne vertébrale (10) comprenant au moins deux éléments d'assujettissement chirurgicaux (16) et au moins un élément de liaison (26), **caractérisé en ce qu'**au moins l'un des au moins deux éléments d'assujettissement chirurgicaux (16) est agencé sous forme d'un élément d'assujettissement (16) selon l'une des revendications précédentes.

15. Système de stabilisation de la colonne vertébrale selon la revendication 14, **caractérisé en ce qu'**il comprend une douille de guidage (126 ; 126') qui peut être couplée de manière temporaire avec le segment de maintien (22) et présente un filet interne (134 ; 134') qui correspond au filet externe (52 ; 52') de l'élément de fixation (42 ; 42').
